# EUROPEAN PATENT APPLICATION

(11) **EP 3 546 578 A1**
(43) Date of publication of application: **02.10.2019**
(21) Application number: 18164538.3
(22) Date of filing: 28.03.2018
(51) Int. Cl.: C12N 15/113, A61K 31/713

(54) **AP-1 BINDING POLYNUCLEOTIDES**

(71) Applicant: Universität Heidelberg, 69117 Heidelberg (DE)
(72) Inventor: Hecker, Markus, 69118 Heidelberg (DE); Wagner, Andreas, 69115 Heidelberg (DE); Remes, Anca, 24116 Kiel (DE); Müller, Oliver, 24226 Heikendorf (DE); Jungmann, Andreas, 69151 Neckargemünd (DE); Katus, Hugo, 69120 Heidelberg (DE); Kallenbach, Klaus, 8366 Hagen (LU); Arif, Rawa, 69120 Heidelberg (DE); Zaraski, Marcin, 69115 Heidelberg (DE)
(74) Representative: Altmann Stößel Dick Patentanwälte PartG mbB

(57) **Abstract**

The present invention relates to an expression construct encoding a short-hairpin activator protein-1 binding polynucleotide (shAP-1 binding polynucleotide), said shAP-1 binding polynucleotide comprising an activator protein 1 (AP-1) binding site sequence and a reverse complement of said AP-1 binding site sequence separated by a spacer sequence. Moreover, the present invention relates to a polyribonucleotide comprising an AP-1 binding site sequence and a reverse complement of said AP-1 binding site sequence separated by a spacer sequence, preferably produced or producible from the expression construct, and to viral particles, compositions, and uses related thereto.

## Description

The present invention relates to an expression construct encoding a short-hairpin activator protein 1 binding polynucleotide (shAP-1 binding polynucleotide), said shAP-1 binding polynucleotide comprising an activator protein 1 (AP-1) binding site sequence and a reverse complement of said AP-1 binding site sequence separated by a spacer sequence. Moreover, the present invention relates to a polyribonucleotide comprising an AP-1 binding site sequence and a reverse complement of said AP-1 binding site sequence separated by a spacer sequence, preferably produced or producible from the expression construct, and to viral particles, compositions, and uses relates thereto.

The rare genetic disorder (2 in 10,000 individuals) Marfan syndrome is *inter alia* characterized by aortic aneurysm, thickening and prolapse of the mitral valve and dilated cardiomyopathy (Pyeritz RE (2017) Ann Cardiothorac Surg 6:595). All these cardiovascular complications can be traced back to (various) loss-of-function mutations in the gene *(FBN1)* encoding fibrillin-1. Fibrillin-1 is a 350 kDa glycoprotein that forms the structural backbone of the elastic and nonelastic microfibrils of extracellular matrix (ECM) in the blood vessel wall and the connective tissue throughout the body. Moreover, it is a structural substrate for elastin deposition and assembly during development and can trap transforming growth factor-β (TGF-β) in its inactive latent form. Therefore, mutations in the *FBN1* gene result in inadequate elastin assembly in the ECM, and inadequate TGF-β mediated activation of matrix metalloproteinase (MMP) activity in the vascular smooth muscle cells and endothelium-dependent inflammation in the wall of the aorta that eventually leads beyond elastin strand breaks (elastolysis) to a general weakening of the ECM and hence aortic dilatation and/or dissection and ultimately to life-threatening aortic aneurysm. There is no cure for the disease and therapeutically delaying the onset of aneurysm formation by β-blockers such as atenolol or angiotensin type 1 receptor blockers such as losartan have proved to be essentially ineffective.

Expression of MMPs is regulated, among others, by the transcription factor activator protein-1 (AP-1), e.g. upon TGF-β stimulation of vascular smooth muscle cells, which binds to the promoter of various MMP genes, but is also involved in regulation of inflammatory processes in mammalian cells. In accordance, inhibition of AP-1 by decoy oligodeoxynucleotides was found to limit restenosis after percutaneous transluminal coronary angioplasty (Buchwald et al. (2002), J Am Coll Cardiol 39:732), was proposed for antiproliferative gene therapy (Park et al. (2005), Gene Ther Mol Biol 9:15), and was found to reduce aortic elastolysis in a murine model of Marfan syndrome (Arif et al. (2017), Nucleic Acids 9:69).

Therapy of Marfan syndrome typically includes conservative and restauratory measures, such as controlling blood pressure and aortic root or mitral valve replacement. However, measures effectively delaying disease progression are still lacking.

Thus, efficient therapies to combat maladaptive processes caused by extracellular matrix insufficiency, e.g. in Marfan syndrome, or by inflammatory processes in blood vessel walls, both of which are mediated by AP-1, are highly required, in particular therapies permitting long-term treatment and/or prevention of the process or its progression. It is therefore an objective of the present invention to provide means and methods to comply with the aforementioned needs, avoiding at least in part the shortcomings of the prior art. This problem is solved by compounds, methods, and uses of the present invention. Embodiments, which might be realized in an isolated fashion or in any arbitrary combination, are listed in the dependent claims.

Accordingly, the present invention relates to an expression construct encoding a short-hairpin activator protein-1 binding polynucleotide (shAP-1 binding polynucleotide), said shAP-1 binding polynucleotide comprising an AP-1 binding site sequence and a reverse complement of said AP-1 binding site sequence separated by a spacer sequence.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may all refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

As used herein, the term "standard conditions", if not otherwise noted, relates to IUPAC standard ambient temperature and pressure (SATP) conditions, i.e. preferably, a temperature of 25°C and an absolute pressure of 100 kPa; also preferably, standard conditions include a pH of 7. Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1%, most preferably less than 0.1% by weight of non-specified component(s). In the context of nucleic acid sequences, the term "essentially identical" indicates a percent identity value of at least 80%, preferably at least 90%, more preferably at least 98%, most preferably at least 99%. As will be understood, the term essentially identical includes 100% identity. The aforesaid applies to the term "essentially complementary" mutatis mutandis. Unless otherwise noted, amino acid and nucleotide symbols are those of WIPO standard ST.25.

The term "polynucleotide" is understood by the skilled person and, preferably, refers to a linear or circular nucleic acid molecule. Preferably, the polynucleotide is RNA or is DNA, including cDNA. The term encompasses single as well as partially or completely doublestranded polynucleotides. In accordance, the term "polyribonucleotide" relates to a polynucleotide consisting of ribonucleotides, i.e. preferably, is RNA. As will be understood by the skilled person, biosynthesis of RNA includes uridine ribonucleotides instead of thymidine ribonucleotides; thus, in polyribonucleotides synthesized by biosynthesis, the "T" residues in the sequences indicated herein would be replaced by "U" residues; thus, e.g. SEQ ID NO:1 would read UGASUCA, with S being C or G, preferably C.

Moreover, comprised are also chemically modified polynucleotides including naturally occurring modified polynucleotides such as glycosylated or methylated polynucleotides or artificially modified derivatives such as biotinylated polynucleotides or polynucleotides comprising phosphorothioates. The polynucleotide of the present invention shall be provided, preferably, either as an isolated polynucleotide (i.e. isolated from its natural context) or in genetically modified form. Preferably, the polynucleotide has a length of at most 1 Mb, more preferably at most 100 kb, even more preferably at most 10 kb, still more preferably at most 1 kb, most preferably at most 100 bp. Preferably, the polynucleotide is a non-naturally occurring polynucleotide; thus, preferably, the nucleotide is an artificial polynucleotide. Also preferably, the polynucleotide is a chimeric polynucleotide; more preferably, the polynucleotide comprises at least one nucleic acid sequence heterologous to the remaining nucleic acid sequences it comprises.

As used herein, the term polynucleotide, preferably, includes variants of the specifically indicated polynucleotides. More preferably, the term polynucleotide relates to polynucleotides essentially identical to the specific polynucleotides indicated. Most preferably, the term polynucleotide relates to the specific polynucleotides indicated. The term "polynucleotide variant", as used herein, relates to a variant of a polynucleotide related to comprising a nucleic acid sequence characterized in that the sequence can be derived from the aforementioned specific nucleic acid sequence by at least one nucleotide substitution, addition and/or deletion, wherein the polynucleotide variant shall have the biological activity or activities as specified for the specific polynucleotide. Thus, it is to be understood that a polynucleotide variant as referred to in accordance with the present invention shall have a nucleic acid sequence which differs due to at least one nucleotide substitution, deletion and/or addition. Preferably, said polynucleotide variant comprises an ortholog, a paralog or another homolog of the specific polynucleotide or of a functional subsequence thereof, e.g. of an AP-1 binding site sequence. Also preferably, said polynucleotide variant comprises a naturally occurring allele of the specific polynucleotide or of a functional subsequence thereof. Polynucleotide variants also encompass polynucleotides comprising a nucleic acid sequence which is capable of hybridizing to the aforementioned specific polynucleotides or functional subsequences thereof, preferably, under stringent hybridization conditions. These stringent conditions are known to the skilled worker and can be found in standard textbooks. A preferred example for stringent hybridization conditions are hybridization conditions in 6x sodium chloride/sodium citrate (= SSC) at approximately 45°C, followed by one or more wash steps in 0.2x SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1x to 5x SSC (pH 7.2). If organic solvent is present in the abovementioned buffer, for example 50% formamide, the temperature under standard conditions is approximately 42°C. The hybridization conditions for DNA:DNA hybrids are preferably for example 0.1x SSC and 20°C to 45°C, preferably between 30°C and 45°C. The hybridization conditions for DNA:RNA hybrids are preferably, for example, 0.1x SSC and 30°C to 55°C, preferably between 45°C and 55°C. The abovementioned hybridization temperatures are determined for example for a nucleic acid with approximately 100 bp (= base pairs) in length and a G+C content of 50% in the absence of formamide; accordingly, other conditions more suitable for low-G+C DNA, which are in principle known to the skilled person, may be found to be more appropriate by the skilled person. The skilled worker knows how to determine the hybridization conditions required by referring to standard textbooks. Alternatively, polynucleotide variants are obtainable by PCR-based techniques such as mixed oligonucleotide primer-based amplification of DNA, e.g. using degenerated primers. As a template, DNA or cDNA from bacteria, fungi, plants or, preferably, from animals may be used. Further, variants include polynucleotides comprising nucleic acid sequences which are at least 70%, at least 75%, at least 80%, at least 85%, at least 90%, at least 95%, at least 98% or at least 99% identical to the specifically indicated nucleic acid sequences or functional subsequences thereof. The percent identity values are, preferably, calculated over the entire nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Feng & Doolittle (1987), J. Mol. Evolution 25: 351, Higgins et al. (1989), CABIOS 5:151) or the programs Gap and BestFit (Needleman and Wunsch (1970), J. Mol. Biol. 48:443) and Smith and Waterman (1981), Adv. Appl. Math. 2: 482-489), are preferably used. Preferably, said programs are used with their standard parameters. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

A polynucleotide comprising a fragment of any of the specifically indicated nucleic acid sequences, said polynucleotide retaining the indicated activity or activities, is also encompassed as a variant polynucleotide of the present invention. A fragment as meant herein, preferably, comprises at least 30, preferably at least 40, more preferably at least 50 consecutive nucleotides of any one of the specific nucleic acid sequences and still has the indicated activity. The polynucleotides of the present invention either consist of, essentially consist of, or comprise the aforementioned nucleic acid sequences. Thus, they may contain further nucleic acid sequences as well. Specifically, the polynucleotide of the present invention may encode e.g. polypeptides, including fusion polypeptides and selectable markers. Such fusion polypeptides may comprise as additional part polypeptides for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and are described elsewhere herein. Selectable markers are known in the art and include in particular antibiotic resistance genes, selectable metabolic markers, e.g. an auxotrophy marker, and the like.

Preferably, the polynucleotide further comprises at least one sequence mediating packaging of said polynucleotide into a viral particle; thus, preferably, the polynucleotide can preferably be provided packaged into a viral particle. Preferably, said viral particle is a replication-incompetent viral particle, e.g. a virus-like particle (VLP). Packaging sequences for relevant viruses are well-known in the art. Preferably, the virus is an adeno-associated virus (AAV), an adenovirus, a retrovirus, preferably a HIV-derivative, or a herpesvirus. Preferably, the virus is an adeno-associated virus; thus, preferably, the viral particle is an adeno-associated virus-like particle (AA-VLP). AA-VLPs are known in the art, including derivatives having a modified cellular tropism compared to wildtype AAV.

The term "short-hairpin activator protein 1 binding polynucleotide", also referred to as "shAP-1 binding polynucleotide", as "hpAP-1 decoy oligodeoxynucleotide", or as "hpAP-1 decoy ODN", as used herein, relates to a polynucleotide comprising an AP-1 binding site sequence and a reverse complement of said AP-1 binding site sequence separated by a spacer sequence, all as specified elsewhere herein; moreover, the shAP-1 binding polynucleotide has the biological activity of binding at least one AP-1 molecule as specified herein below. The activity of a polynucleotide of binding at least one AP-1 molecule can be established by methods known in the art and as described herein in Hecker et al. (2008). Decoy oligodeoxynucleotides to treat inflammatory diseases. (book chapter) In: Therapeutic Oligonucleotides; (ed. Kurreck J) RSC Publishing, Cambridge, U.K., p. 163. Preferably, the dissociation constant K_{d} of the AP-1/shAP-1 binding polynucleotide complex is at most 10⁻⁶ M, more preferably at most 10⁻⁷ M, even more preferably at most 10⁻⁸ M, most preferably at most 10⁻⁹ M in case the shAP-1 binding polynucleotide is a DNA, and is at most 10⁻⁵ M, more preferably at most 10⁻⁶ M, even more preferably at most 10⁻⁷ M, most preferably at most 10⁻⁸ M in case the shAP-1 binding polynucleotide is an RNA. Preferably, the binding of the shAP-1 binding polynucleotide to the AP-1 polypeptide is specific; thus, preferably, the binding of the shAP-1 binding polynucleotide to a polypeptide which is not an AP-1 as specified preferably is significantly weaker than binding of the polynucleotide to an AP-1. More preferably, the K_{d} of a complex of the shAP-1 binding polynucleotide and a non-AP-1 transcription factor is at least 10fold, more preferably at least 100fold, even more preferably at least 1000fold higher than the K_{d} for the AP-1/polynucleotide complex. Preferably, the shAP-1 binding polynucleotide comprises at least one of SEQ ID NOs: 1 to 10, as shown in Table 1.

**Table 1: AP-1 binding sequences**

| **Sequence** | **SEQ ID NO** |
|---|---|
| TGASTCA | 1 |
| GTGCTGACTCAGCAC | 2 |
| TGTGCTGACTCAGCACA | 3 |
| TTGTGCTGACTCAGCACAA | 4 |
| CGCTTGATGACTCAGCCGGAA | 5 |
| GTCGCTTAGTGACTAAGCGAC | 6 |
| TTGCTGACTCATGAGTCAGCAA | 7 |
| CTGCGGTGCTGACTCAGCACG | 8 |
| CGTGCTGAGTCAGCACCGCAG | 9 |
| CTGCGGTGCTGACTCAGCACGAAACGTGCTGAGTCAGCACCGCAG | 10 |

More preferably, the shAP-1 binding polynucleotide comprises a nucleic acid sequence corresponding to at least one of the consensus sequences of SEQ ID NO: 1. As is understood by the skilled person, a nucleic acid sequence corresponding to a consensus sequence may be realized by providing a polynucleotide comprising one of the specific sequences represented by the consensus sequence, by providing a polynucleotide comprising one or more nucleotides pairing with more than one nucleotide, e.g. inosine, by providing a mixture of polynucleotides each comprising one of the specific sequences represented by the consensus sequence, or a combination of the aforesaid. More preferably, the shAP-1 binding polynucleotide comprises at least one of SEQ ID NOs: 2 to 9. Most preferably, the shAP-1 binding polynucleotide comprises, preferably consists of, the sequence of SEQ ID NO: 10 or a sequence at least 75% identical thereto. Preferably, said sequence at least 75% identical to SEQ ID NO: 10 comprises at least one AP-1 binding sequence as specified elsewhere herein. More preferably, the shAP-1 binding polynucleotide comprises, preferably consists of, the sequence of SEQ ID NO: 10. As will be understood, the shAP-1 binding polynucleotide, if it is a single-stranded polynucleotide, preferably forms a stem-loop structure in solution, preferably in a cell, wherein the AP-1 binding site sequence and its reverse complement are hybridized to form a double stranded AP-1 binding site sequence.

Preferably, the shAP-1 binding polynucleotide comprises at least one further transcription factor binding site sequence and a corresponding reverse complement of said at least one further transcription factor binding site sequence. In view of the above, it is clear that, preferably, the AP-1 binding site sequence, the further transcription factor binding site sequence and their respective reverse complements are arranged such that the corresponding sequences hybridize under appropriate conditions. E.g. preferably, the 5' to 3' sequence is AP-1 binding site sequence - further transcription factor binding site sequence - spacer sequence - reverse complement of further transcription factor binding site sequence - reverse complement of AP-1 binding site sequence; or is further transcription factor binding site sequence - AP-1 binding site sequence - spacer sequence - reverse complement of AP-1 binding site sequence - reverse complement of further transcription factor binding site sequence. Preferably, the at least one further transcription factor binding site sequence is an AP-1 binding site sequence or is a binding site sequence for at least one further transcription factor. As will be understood, a further AP-1 binding site sequence may be identical to the (first) AP-1 binding site sequence or may be non-identical thereto. Preferably, the further transcription factor is a further transcription factor involved in the same signaling pathway as AP-1, and/or is a transcription factor contributing to the same disease as AP-1.

Preferably, the shAP-1 binding polynucleotide is a DNA. The DNA shAP-1 binding polynucleotide preferably consists of or essentially consists of the AP-1 binding site sequence and a reverse complement of said AP-1 binding site sequence separated by a spacer sequence as specified herein. Also preferably, the shAP-1 binding polynucleotide is an RNA. Preferably, said RNA is a short-hairpin RNA, preferably expressed or expressible from an expression construct as specified herein.

The term "expression construct", as used herein, relates to a polynucleotide encoding a shAP-1 binding polynucleotide and comprising at least one nucleic acid sequence causing the shAP-1 binding polynucleotide to be expressed in a host cell. Preferably, in case the expression construct is a DNA, the nucleic acid sequence causing the shAP-1 binding polynucleotide to be expressed preferably is a promoter, more preferably an RNA-polymerase promoter. The skilled person is able to select an appropriate promoter according to purpose; preferably, the promoter is an inducible promoter, more preferably, the promoter is a constitutive promoter. Also preferably, the promoter is a cell-type specific promoter, in particular an endothelial cell-specific promoter or a vascular smooth muscle cell-specific promoter. Also preferably, in case the expression construct is an RNA, the expression construct comprises at least one reverse transcriptase initiation site, i.e. preferably, from the RNA expression construct a DNA polynucleotide as specified above is expressed in a host cell comprising a reverse transcriptase.

The terms "activator protein-1" and its abbreviation "AP-1" are known to the skilled person. AP-1 is a transcription factor involved in the cellular response to a variety of stimuli, including cytokine and growth factor response, stress, infections, and the like. As referred to herein, AP-1 is a heterodimer of polypeptides independently selected from the c-Fos family, c-Jun family, ATF family, and JDP family of proteins. Preferably, AP-1 is human AP-1. Preferably, AP-1 comprises at least one of a c-Jun and a c-Fos polypeptide; more preferably, AP-1 is a heterodimer of c-Jun and c-Fos, most preferably, AP-1 is a heterodimer of human c-Jun (Genbank Acc No: BC068522.1) and human c-Fos (Genbank Acc No: V01512.1).

The term "AP-1 binding site sequence", as used herein, relates to a nucleic acid sequence which, when comprised in a polynucleotide, causes at least one AP-1 to bind to said polynucleotide at said nucleic acid sequence. Preferably, the AP-1 binding site sequence comprises the sequence TGASTCA (SEQ ID NO: 1), wherein S is G or C. More preferably, the AP-1 binding site sequence comprises the sequence GTGCTGACTCAGCAC (SEQ ID NO: 2); preferably comprises the sequence TGTGCTGACTCAGCACA (SEQ ID NO: 3); TTGTGCTGACTCAGCACAA (SEQ ID NO: 4); CGCTTGATGACTCAGCCGGAA (SEQ ID NO: 5),; GTCGCTTAGTGACTAAGCGAC (SEQ ID NO: 6) or TTGCTGACTCATGAGTCAGCAA (SEQ ID NO: 7). Even more preferably, the AP-1 binding site sequence comprises the sequence CTGCGGTGCTGACTCAGCACG (SEQ ID NO: 8) or a sequence at least 75% identical thereto, preferably comprises the sequence CGTGCTGAGTCAGCACCGCAG (SEQ ID NO: 9). Preferably, the sequence at least 75% identical to SEQ ID NO: 8 comprises the sequence TGASTCA (SEQ ID NO: 1), wherein S is G or C, as specified above. Preferably, the AP-1 binding site sequence has a length of from 7 to 22 nucleotides, preferably 7 to 11 nucleotides.

The term "reverse complement" of a nucleic acid sequence is understood by the skilled person. The term relates to a nucleic acid comprising the complementary bases to the original sequence in reverse order with respect to the 5' to 3' orientation of a polynucleotide, such that a single-stranded polynucleotide consisting of a given sequence and a single-stranded polynucleotide consisting of its reverse complement will hybridize over their respective complete lengths under appropriate conditions. Thus, e.g. the reverse complement of the sequence 5'-TGASTCA-3' is 5'-ACTSAGT-3'.

The term "spacer sequence", as used herein, relates to a nucleic acid neither being part of the AP-1 binding site sequence nor of its reverse complement. Thus, preferably, the spacer sequence, when comprised in a polynucleotide as specified herein, hybridizes neither to the AP-1 binding site sequence nor to its reverse complement. More preferably, the spacer sequence is a single-stranded sequence after the AP-1 binding site sequence and its reverse complement have hybridized in the polynucleotide. Thus, preferably, the polynucleotide forms a stem-loop structure, wherein the spacer sequence is the sequence comprised in the loop. Preferably, the spacer sequence has a length of from 3 to 25 nucleotides, preferably 3 to 15 nucleotides, more preferably 3 to 10 nucleotides.

Advantageously, it was found in the work underlying the present invention that stem-loop structures expressed in a cell can be used analogously to binding oligonucleotides. Moreover, it was surprisingly found that RNA stem-loop structures comprising appropriate sequences function as binding sequence (decoys) for AP-1. Moreover, it was found that using these stem-loop structures, AP-1 signaling and, thus, deleterious effects caused by such signaling, can be diminished or interrupted.

The definitions made above apply mutatis mutandis to the following. Additional definitions and explanations made further below also apply for all embodiments described in this specification mutatis mutandis.

The present invention further relates to a polyribonucleotide comprising an activator protein 1 (AP-1) binding site sequence and a reverse complement of said AP-1 binding site sequence separated by a spacer sequence, preferably produced or producible from the expression construct according to the present invention.

The present invention further relates to a expression construct according to the present invention and/or a polyribonucleotide according to the present invention for use in treating, preventing, and/or preventing progression of disease.

The present invention also relates to an expression construct according to the present invention and/or a polyribonucleotide according to the present invention for use in treating, preventing, and/or preventing progression of disease caused by extracellular matrix insufficiency and/or inflammation within the blood vessel wall (arteries and veins).

The terms "treating" and "treatment" refer to an amelioration of the diseases or disorders referred to herein or the symptoms accompanied therewith, preferably to a significant extent. Said treating as used herein also includes an entire restoration of health with respect to the diseases or disorders referred to herein. It is to be understood that treating, as the term is used herein, may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well-known statistic evaluation tools, e.g., determination of confidence intervals, p-value determination, Student's t-test, Mann-Whitney test etc. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 10%, at least 20%, at least 50%, at least 60%, at least 70%, at least 80%, or at least 90% of the subjects of a given cohort or population. As will be understood by the skilled person, effectiveness of treatment is dependent on a variety of factors including, e.g. severity of existing disease, accompanying diseases, and further risk factors. Preferably, treating comprises decreasing inappropriate activation of AP-1 in affected cells. Preferably, treating a disease or condition with a compound recited in this specification consists of a single administration of said compound within a long period of time, preferably six months, more preferably one year, most preferably two years, i.e., preferably, is a long-term treatment. More preferably, treating a disease or condition with a compound recited in this specification consists of a single administration of said compound, i.e., preferably, is a one-time treatment. The treatment, preferably, includes additional therapeutic, or preventive measures.

The tem "preventing" refers to retaining health with respect to the diseases or disorders referred to herein for a certain period of time in a subject. It will be understood that said period of time may be dependent on the amount of drug compound which has been administered and on individual factors of the subject discussed elsewhere in this specification. It is to be understood that prevention may not be effective in all subjects treated with the compound. However, the term requires that, preferably, a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well-known statistic evaluation tools discussed elsewhere in this specification. Preferably, preventing a disease or condition with a compound recited in this specification consists of a single administration of said compound, in particular to a subject at risk for developing said disease or condition, within a long period of time, preferably six months, more preferably one year, most preferably two years, i.e., preferably, is a long-term preventive treatment. More preferably, preventing a disease or condition with a compound recited in this specification consists of a single administration of said compound, i.e., preferably, is a one-time preventive treatment.

As will be understood by the skilled person, some of the diseases or disorders referred to herein are inherited diseases, e.g. Marfan syndrome, which cannot be prevented by the means and methods of the present invention and which may not be amenable to treatment leading to an improvement, meaning that only conservative treatment may be possible. Thus, the term "preventing progression" refers to preventing further aggravation of the diseases or disorders referred to herein or the symptoms accompanied therewith for a certain period of time in a subject. It will be understood that said period of time may be dependent on the amount of drug compound which has been administered and on individual factors of the subject discussed elsewhere in this specification. It is to be understood that preventing progression may not be effective in all subjects treated with the compound. However, the term requires that, preferably, a statistically significant portion of subjects of a cohort or population are effectively prevented from aggravation of a disease or disorder referred to herein or its accompanying symptoms. Preferably, a cohort or population of subjects is envisaged in this context which normally, i.e. without preventive measures according to the present invention, would show aggravation of a disease or disorder as referred to herein. Whether a portion is statistically significant can be determined without further ado by the person skilled in the art using various well-known statistic evaluation tools discussed elsewhere in this specification. Preferably, preventing progression of a disease or condition with a compound recited in this specification consists of a single administration of said compound, in particular to a subject at risk for developing said disease or condition, within a long period of time, preferably six months, more preferably one year, most preferably two years, i.e., preferably, is a long-term preventive treatment. More preferably, preventing progression of a disease or condition with a compound recited in this specification consists of a single administration of said compound, i.e., preferably, is a one-time preventive treatment.

The term "disease caused by extracellular matrix insufficiency", as used herein, relates to any disease or symptom caused or aggravated by and/or correlating with insufficient stability of the extracellular matrix. Preferably, the disease caused by extracellular matrix insufficiency is Marfan syndrome. Preferably, the disease caused by extracellular matrix insufficiency is caused by a mutation in a fibrillin gene, preferably the fibrillin-1 gene. more preferably the human fibrillin-1 gene. Preferably, said mutation is a mutation associated with Marfan syndrome. Preferably, treating, preventing, and/or preventing progression of the disease caused by extracellular matrix insufficiency comprises decreasing expression of at least one matrix metalloproteinase (MMP), preferably of MMP2 and/or MMP9.

The term "inflammation within the blood vessel wall " is understood by the skilled person to relate to any inflammatory process involving a wall of a blood vessel. In accordance, the term "disease caused by inflammation within the blood vessel wall " relates to any disease or disorder associated with an inflammatory process involving a wall of a blood vessel. Preferably, the disease caused by inflammation within the blood vessel wall is chronic graft rejection, venous bypass vasculopathy, restenosis after angioplasty, preferably percutaneous transluminal angioplasty with or without stent placement, and/or varicose veins.

The present invention also relates to a viral particle comprising an expression construct according to the present invention and/or a polyribonucleotide according to the present invention. Preferably, the viral particle is a viral particle as specified herein above, more preferably a VLP as specified herein above. Thus, preferably, the viral particle is an adeno-associated VLP (AAV-VLP), preferably is an AAV9-VLP.

The present invention further relates to a method for treating, preventing, and/or preventing progression of a disease caused by extracellular matrix insufficiency and/or inflammation within the blood vessel wall in a subject suffering therefrom or expected to suffer therefrom comprising
a) administering an effective dose of an expression construct according to the present invention and/or a polyribonucleotide according to the present invention, and/or a viral particle according to the present invention to said subject; and, thereby,
b) treating, preventing, and/or preventing progression of a disease caused by extracellular matrix insufficiency and/or inflammation within the blood vessel wall.

The method for treating, preventing, and/or preventing progression according to the present invention, preferably, is an in vivo method. Moreover, it may comprise steps in addition to those explicitly mentioned above. For example, further steps may relate, e.g., to providing an expression construct according to the present invention for step a), or additional treatment of the disease in step b). Moreover, one or more of said steps may be performed by automated equipment.

As used herein, the term "subject" relates to a vertebrate. Preferably, the subject is a mammal, more preferably, a mouse, rat, cat, dog, hamster, guinea pig, sheep, goat, pig, cattle, or horse. Still more preferably, the subject is a primate. Most preferably, the subject is a human. Preferably, the subject is afflicted with or having an increased risk of becoming afflicted with a disease related to herein. Preferably, the subject is a graft recipient, a venous bypass recipient, suffers from varicose veins or is subjected to (percutaneous transluminal) angioplasty with or without stent placement. More preferably, the subject suffers from Marfan syndrome, even more preferably was diagnosed to have a mutation in the fibrillin-1 gene.

Preferably, the method for treating, preventing, and/or preventing progression comprises topical and/or systemic application of a polynucleotide and/or a viral particle as specified herein. Systemic application, preferably, comprises transcutaneous, intraarterial, or intravenous application. As will be understood by the skilled person, the preferred mode of administration will depend on the type of disease and, thus, the target tissue. Preferably, intraarterial or intravenous application is catheter-assisted. It is however, also envisaged that the method for treating, preventing, and/or preventing progression comprises systemic administration of the polynucleotide and/or the viral particle, e.g. by intravenous infusion. Preferably, in particular in case the subject is a graft recipient or the recipient of a bypass graft, the graft is contacted with the composition during the time of warm ischemia.

The present invention further relates to a composition comprising an expression construct according to the present invention, a polyribonucleotide according to the present invention, and/or a viral particle according to the present invention, and a carrier.

The term "composition", as used herein, relates to a mixture of compounds comprising at least a polyribonucleotide, expression vector, and/or viral particle as specified herein and at least one carrier. The carrier(s) must be acceptable in the sense of being compatible with the other ingredients of the composition and being not deleterious to a potential recipient thereof. The carrier employed may be, for example, a gel or a liquid. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Suitable carriers comprise those mentioned above and others well known in the art. The carrier(s) is/are selected so as not to affect the biological activity of the composition. Examples of such diluents are distilled water, physiological saline, Ringer's solutions, dextrose solution, and Hank's solution. Preferably, in the composition, at least 50%, preferably at least 75%, more preferably at least 90%, even more preferably at least 95%, most preferably essentially all viral particles are viral particles comprising a polynucleotide as specified elsewhere herein.

Preferably, the composition is a pharmaceutical composition; thus, preferably, the carrier is a pharmaceutically acceptable carrier. In addition, the pharmaceutical composition or formulation may also include other carriers, adjuvants, or nontoxic, nontherapeutic, non-immunogenic stabilizers and the like. The pharmaceutical compositions are, preferably, administered topically or systemically. Suitable routes of administration conventionally used for drug administration are topical, intravenous, or parenteral administration as well as inhalation. However, depending on the nature and mode of action of a compound, the pharmaceutical compositions may be administered by other routes as well, in particular as specified elsewhere herein. Moreover, the polynucleotide can be administered in combination with other drugs either in a common pharmaceutical composition or as separated pharmaceutical compositions wherein said separated pharmaceutical compositions may be provided in form of a kit of parts. The binding polypeptide is, preferably, administered in conventional dosage forms prepared by combining the drugs with standard pharmaceutical carriers according to conventional procedures. These procedures may involve mixing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

A therapeutically effective dose refers to an amount of the polynucleotide to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED₅₀ (the dose therapeutically effective in 50% of the population) and LD₅₀ (the dose lethal to 50% of the population). The dose ratio between therapeutic and toxic effects is the therapeutic index, and it can be expressed as the ratio, LD₅₀/ED₅₀. The dosage regimen will be determined by the attending physician and other clinical factors; preferably in accordance with any one of the above described methods. As is well known in the medical arts, dosages for any one patient depend upon many factors, which may include the patient's size, body surface area, age, the particular compound to be administered, sex, time and route of administration, general health, and other drugs being administered concurrently. Progress can be monitored by periodic assessment. A typical dose can be, for example, in the range of 1 to 1000 µg; however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 1 mg units per kilogram of body weight per minute, respectively. Preferably, the pharmaceutical composition is administered once to the subject, i.e., preferably, is used as a one-time treatment. Thus, preferably, treating or preventing a disease or condition with a compound recited in this specification consists of a single administration of said compound. Depending on the subject and the mode of administration, the quantity of substance administration may vary over a wide range to provide from about 0.01 mg per kg body mass to about 10 mg per kg body mass. As will be appreciated, in case a vector is used, e.g. an AAV vector, quantity of substance administration may be even lower, e.g. from 1 ng to 1 mg per dose, preferably as a one-time dose. Preferably, in case the compound of the present invention is administered packaged in a viral vector, the dose is of from 10¹⁰ to 10¹⁴ viral particles per administration. The pharmaceutical compositions and formulations referred to herein are administered at least once in order to treat or ameliorate or prevent a disease or condition recited in this specification. However, the said pharmaceutical compositions may be administered more than one time, for example from one to four times. Progress can be monitored by periodic assessment.

Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent. The resulting formulations are to be adapted to the mode of administration, i.e. in the forms of tablets, capsules, suppositories, solutions, suspensions or the like. Dosage recommendations shall be indicated in the prescribers or user's instructions in order to anticipate dose adjustments depending on the considered recipient.

The present invention further relates to the use of an expression construct according to the present invention, a polyribonucleotide according to the present invention, and/or a viral particle according to the present invention for inhibiting at least AP-1; and the present invention relates to the use of a expression construct according to the present invention, a polyribonucleotide according to the present invention, and/or a viral particle according to the present invention in the manufacture of a pharmaceutical composition for treating AP-1-mediated disease.

The term "host cell", as used herein, relates to a eukaryotic cell, preferably a vertebrate cell, more preferably a mammalian cell, most preferably, a human cell. Preferably, the host cell comprises at least one polynucleotide polymerase, preferably a reverse transcriptase and/or an RNA polymerase. More preferably, said polynucleotide polymerase initiates polymerization at a nucleic acid sequence comprised in the expression construct, preferably at the reverse transcriptase initiation site and/or the promoter.

In view of the above, the following embodiments are particularly envisaged:
1. An expression construct encoding a short-hairpin activator protein-1 binding polynucleotide (shAP-1 binding polynucleotide), said shAP-1 binding polynucleotide comprising an activator protein-1 (AP-1) binding site sequence and a reverse complement of said AP-1 binding site sequence separated by a spacer sequence.
2. The expression construct of embodiment 1, wherein said AP-1 is human AP-1.
3. The expression construct of embodiment 1 or 2, wherein said AP-1 binding site sequence has a length of from 7 to 22 nucleotides, preferably 7 to 10 nucleotides.
4. The expression construct of any one of embodiments 1 to 3, wherein said shAP-1 binding polynucleotide is a short-hairpin RNA (shRNA).
5. The expression construct of any one of embodiments 1 to 4, wherein said shAP-1 binding polynucleotide comprises at least one further transcription factor binding site sequence and a corresponding reverse complement of said at least one further transcription factor binding site sequence.
6. The expression construct of embodiment 5, wherein said at least one further transcription factor binding site sequence is a binding site sequence for AP-1 or is a binding site sequence for at least one further transcription factor.
7. The expression construct of any one of embodiments 1 to 6 further comprising at least one sequence mediating packaging of said expression construct into a viral particle.
8. The expression construct of any one of embodiments 1 to 7, wherein said AP-1 binding site sequence, said reverse complement of said AP-1 binding site sequence, and said spacer sequence are comprised by a polynucleotide derived from an adeno-associated virus.
9. The expression construct of embodiment 8, wherein said polynucleotide derived from an adeno-associated virus is comprised in a virus-like particle (VLP), preferably an adeno-associated VLP.
10. The expression construct of any one of embodiments 1 to 9, wherein said polynucleotide is a recombinant polyribonucleotide.
11. The expression construct of any one of embodiments 1 to 10, wherein said shAP-1 binding polynucleotide has a length of from 30 to 70 nucleotides.
12. The expression construct of any one of embodiments 1 to 11, wherein the spacer sequence has a length of from 3 to 25 nucleotides, preferably 3 to 15 nucleotides, more preferably 3 to 10 nucleotides.
13. The expression construct of any one of embodiments 1 to 12, wherein said AP-1 binding site sequence comprises the sequence TGASTCA (SEQ ID NO:1), with S being C or G.
14. The expression construct of any one of embodiments 1 to 13, wherein said AP-1 binding site sequence comprises the sequence GTGCTGACTCAGCAC (SEQ ID NO: 2); preferably comprises the sequence TGTGCTGACTCAGCACA (SEQ ID NO: 3); TTGTGCTGACTCAGCACAA (SEQ ID NO: 4); CGCTTGATGACTCAGCCGGAA (SEQ ID NO: 5),; GTCGCTTAGTGACTAAGCGAC (SEQ ID NO: 6), TTGCTGACTCATGAGTCAGCAA (SEQ ID NO: 7), CTGCGGTGCTGACTCAGCACG (SEQ ID NO: 8), CGTGCTGAGTCAGCACCGCAG (SEQ ID NO: 9), or a sequence at least 75% identical to any of the above, preferably comprising the sequence of SEQ ID NO: 1.
15. The expression construct of any one of embodiments 1 to 14, wherein said AP-1 binding site sequence comprises the sequence of any one of SEQ ID NOs: 2 to 9, preferably SEQ ID NO:8 and/or SEQ ID NO:9.
16. The expression construct of any one of embodiments 1 to 15, wherein the polyribonucleotide comprises, preferably consists of, the sequence CTGCGGTGCTGACTCAGCACGAAACGTGCTGAGTCAGCACCGCAG (SEQ ID NO:10) or a sequence at least 75% identical thereto, preferably comprising the sequence of SEQ ID NO:1.
17. The expression construct of any one of embodiments 1 to 16 further comprising an RNA polymerase promoter upstream of the shAP-1 binding polynucleotide encoding sequence.
18. The expression construct of any one of embodiments 1 to 17 further comprising a reverse transcriptase initiation site upstream of the shAP-1 binding polynucleotide encoding sequence.
19. A polyribonucleotide comprising an activator protein 1 (AP-1) binding site sequence and a reverse complement of said AP-1 binding site sequence separated by a spacer sequence, preferably produced or producible from the expression construct according to any one of embodiments 1 to 17.
20. An expression construct according to any one of embodiments 1 to 18 and/or a polyribonucleotide according to embodiment 19 for use in treating, preventing, and/or preventing progression of disease.
21. An expression construct according to any one of embodiments 1 to 18 and/or a polyribonucleotide according to embodiment 19 for use in treating, preventing, and/or preventing progression of disease caused by extracellular matrix insufficiency and/or inflammation within the blood vessel wall.
22. The expression construct and/or polyribonucleotide for use of embodiment 21, wherein said disease caused by extracellular matrix insufficiency is Marfan syndrome.
23. The expression construct and/or polyribonucleotide for use of embodiment 22, wherein said extracellular matrix insufficiency is caused by a mutation in a fibrillin gene, preferably the fibrillin-1 gene.
24. The expression construct and/or polyribonucleotide for use of any one of embodiments 21 to 23, wherein said treating, preventing, and/or preventing progression comprises decreasing expression of at least one matrix metalloproteinase (MMP), preferably of MMP2 and/or MMP9.
25. The expression construct and/or polyribonucleotide for use of embodiment 21, wherein said disease caused by inflammation within the blood vessel wall is chronic graft rejection, venous bypass vasculopathy, varicose veins, and/or restenosis after (percutaneous transluminal) angioplasty with or without stent placement.
26. A viral particle comprising an expression construct according to any one of embodiments 1 to 18 and/or a polyribonucleotide according to embodiment 19.
27. The viral particle of embodiment 26, wherein said viral particle is an adeno-associated VLP (AAV-VLP), preferably is an AAV9-VLP.
28. A method for treating, preventing, and/or preventing progression of a disease caused by extracellular matrix insufficiency and/or inflammation within the blood vessel wall in a subject suffering therefrom or expected to suffer therefrom comprising
   a) administering an effective dose of an expression construct according to any one of embodiments 1 to 18, a polyribonucleotide according to embodiment 19, and/or a viral particle according to embodiment 26 or 27 to said subject; and, thereby,
   b) treating, preventing, and/or preventing progression of a disease caused by extracellular matrix insufficiency and/or inflammation within the blood vessel wall.
29. The method of embodiment 28, wherein said disease caused by extracellular matrix insufficiency is Marfan syndrome.
30. The method of embodiment 28 or 29, wherein said Marfan syndrome is caused by a mutation in a fibrillin gene, preferably the fibrillin-1 gene.
31. The method of any one of embodiments 28 to 30 comprising (i) topical and/or systemic application, preferably epicutaneous, transcutaneous, intraarterial, or intravenous application or (ii) application to a graft during the time of warm ischemia, of said polyribonucleotide, expression construct and/or viral particle.
32. A composition comprising an expression construct according to any one of embodiments 1 to 18, a polyribonucleotide according to embodiment 19, and/or a viral particle according to embodiment 26 or 27, and a carrier.
33. A composition comprising the viral particle according to embodiment 26 or 27 and a carrier, wherein at least 50%, preferably at least 75%, more preferably at least 90%, even more preferably at least 95%, most preferably essentially all viral particles are viral particles according to embodiment 26 or 27.
34. The composition of embodiment 32 or 33, wherein the composition is a pharmaceutical composition and wherein said carrier is a pharmaceutically acceptable carrier.
35. Use of an expression construct according to any one of embodiments 1 to 18, a polyribonucleotide according to embodiment 19, and/or a viral particle according to embodiment 26 or 27 for inhibiting, preferably in vitro inhibiting, at least AP-1.
36. Use of an expression construct according to any one of embodiments 1 to 18, a polyribonucleotide according to embodiment 19, and/or a viral particle according to embodiment 26 or 27 in the manufacture of a pharmaceutical composition for treating, preventing and/or preventing progression of disease caused by extracellular matrix insufficiency and/or inflammation within the blood vessel wall.
37. The subject matter of any of the preceding embodiments, wherein said AP-1 binding site lies in the 5'-untranscribed region of at least 50%, more preferably at least 80%, even more preferably at least 90 %, most preferably all, AP-1 target genes.

All references cited in this specification are herewith incorporated by reference with respect to their entire disclosure content and the disclosure content specifically mentioned in this specification.

### Figure Legends

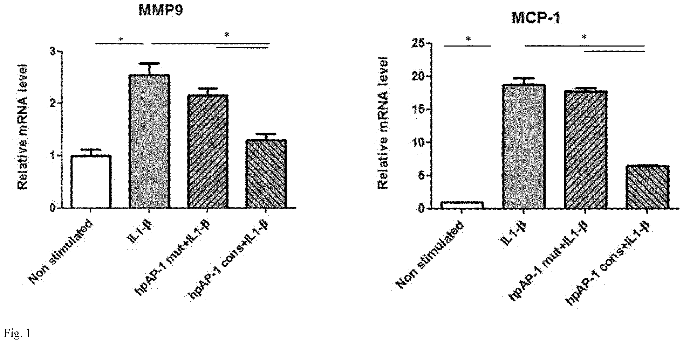

Figure 1: Naked hairpin AP-1 consensus decoy ODN but not the mutant control ODN significantly decreases MMP-9 and MCP-1 mRNA expression in vascular SMCs isolated from aorta of mgR/mgR mice. MMP-9 and MCP-1 mRNA levels were normalised to RPL32 (ribosomal protein L32) as housekeeping gene (n=5, *p<0.05).

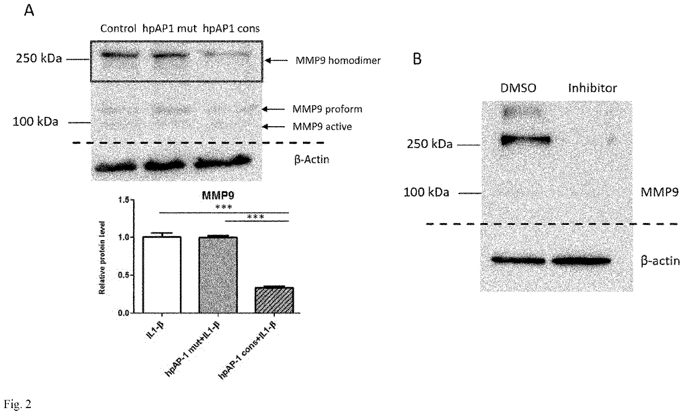

Figure 2: Naked hairpin AP-1 consensus decoy ODN significantly decrease IL-1β-induced MMP-9 protein expression in aortic SMCs. (A) MMP-9 Western blot analysis of whole cell lysates. β-Actin was used as a loading control and the relative rise in protein abundance was normalized to the level attained in IL-1β (20 ng/mL) stimulated SMCs *(n=4,* ***p<0.001). (B) Evaluation of the 250 kDa band detected in the Western blot experiments. By using a small molecule which blocks MMP-9 dimerization, the band could be chanarterized as high molecular weight MMP-9 homodimer.

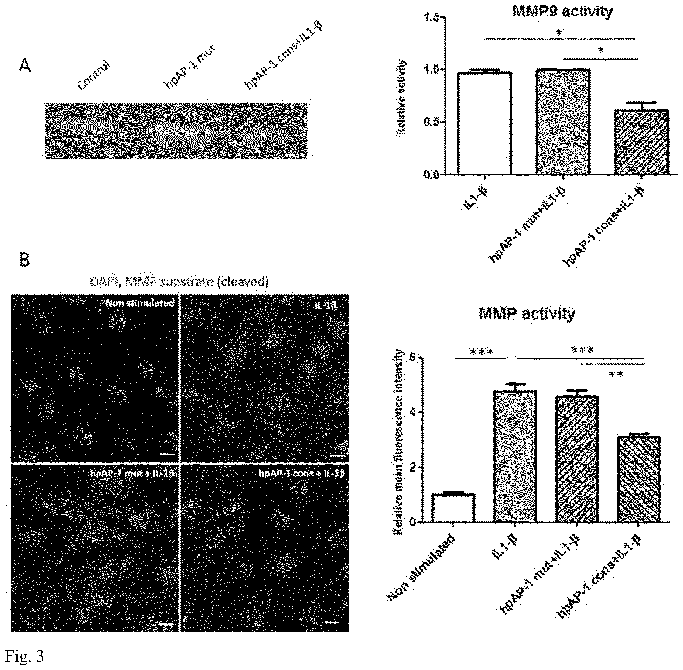

Figure 3: Hairpin AP-1 consensus decoy ODNs decrease IL-1β-induced MMP activity in aortic SMCs. (A) MMP-9 gelatinolytic activity as shown by gel zymography. The graph depicts the densitometric analysis of the bands normalized to IL-1β (20 ng/mL) alone *(n=4,* *p<0.05) (B) Intracellular MMP activity was analysed using a FRET substrate specific for 12 different MMPs. The signal represents the fluorescence generated by cleaved substrate and correlates with the total activity of intracellular MMP. The graph shows the statistical quantification of mean red fluorescence intensity of the indicated treatment groups. Cell nuclei were stained with DAPI. The scale bar represents 75 µm, 20 images were analysed per group (*n*=4, ***p<0.001, **p<0.01).

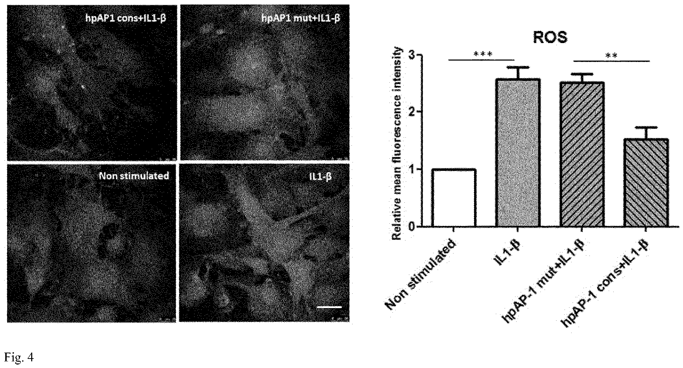

Figure 4: Naked hairpin AP-1 consensus decoy ODN decreases IL-1β-induced ROS production in aortic SMCs. (Left) Representative images showing CM-DCF fluorescence, recorded using confocal microscopy, in the green channel. (Right) Mean fluorescence intensity was analysed and normalized to the level in the non-stimulated control. The scale bar represents 75 µm, 20 images were analysed per group (*n*=4, ***p<0.001, **p<0.01).

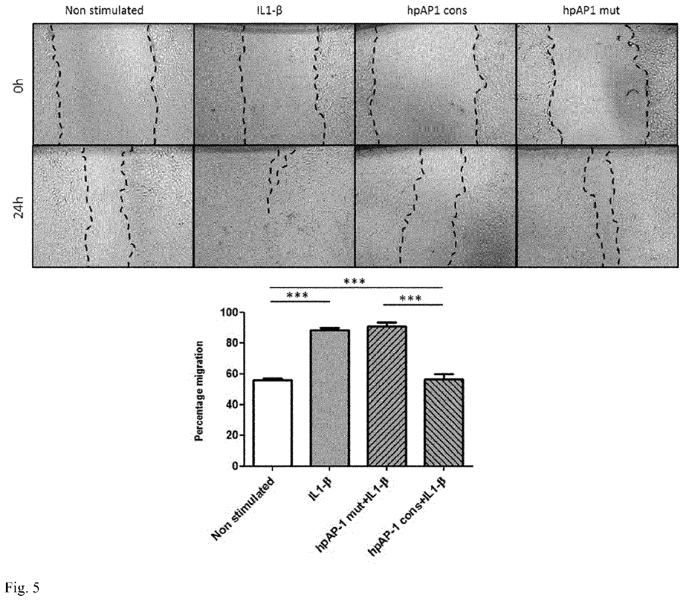

Figure 5: Naked hairpin AP-1 consensus decoy ODN significantly decreases the migration rate of vascular SMCs. (Top) Depicted are representative images of SMCs immediately after removal of the inserts (0 hour), and after 24 hour stimulation with 20 ng/mL IL-1β. (Bottom) Quantitative summary of the relative migration rate in the four treatment groups (*n*=4, ***p<0.001).

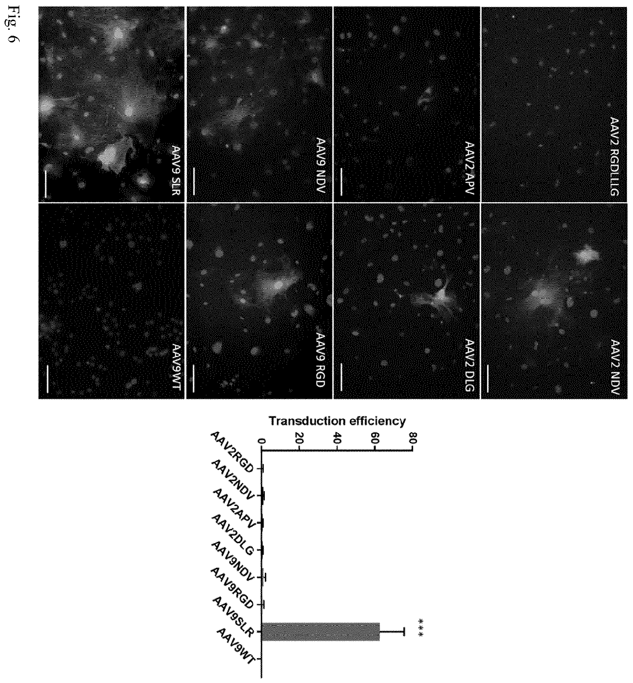

Figure 6: Transduction efficacy of different AAV serotypes with modified capsid. (Left) Representative images of mgR/mgR aortic SMCs 3 days post transduction with the tested adenoassociated viral vectors. EGFP fluorescence (green) was analysed as a marker of transduction efficacy of the different viral vectors and the percentage of transduced cells was calculated (right). Nuclei were stained with DAPI to assess the number of cells per field of view. Scale bar represents 82 µm *(n=4,* ***p< 0.0001, compared to AAV9WT).

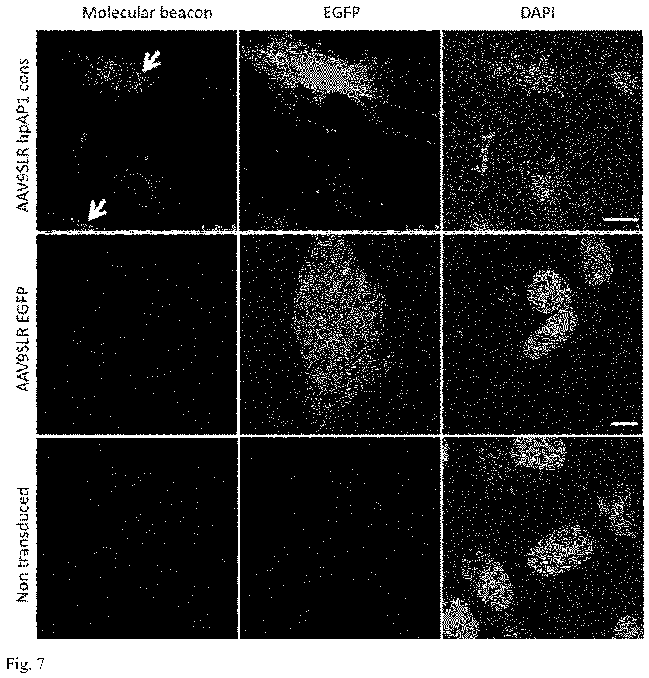

Figure 7: AAV9SLR-mediated expression of the hpAP-1 consensus RNA decoy ODN in vascular SMCs. The bright signal shows hybridization of the molecular beacon to its target, proving expression of the shRNA molecule in the transduced, eGFP-positive cells (green). Non-transduced SMCs as well as AAV9SLR-eGFP treated cells served as controls. DAPI staining (blue) was employed to visualize the nuclei. The scale bar represents 75 µm (*n*=3, exemplary images).

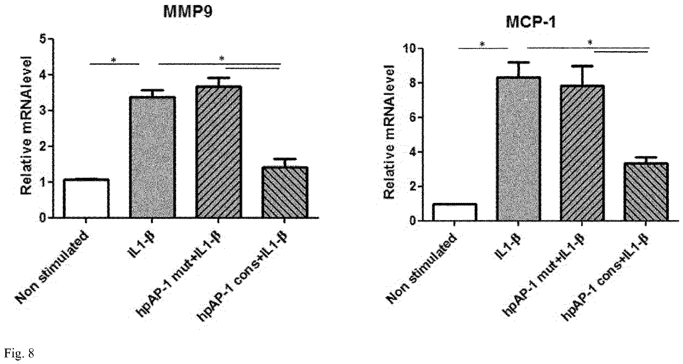

Figure 8: AAV9SLR-mediated expression of the hpAP-1 consensus RNA decoy ODN significantly decreases expression of the AP-1 target genes *MMP9* and *CCL2* (MCP-1) in vascular SMCs. Messenger RNA levels of MMP-9 and MCP-1 were compared to RPL32 as a housekeeping gene. Values were normalized to the non-stimulated controls *(n=4,* *p<0.05).

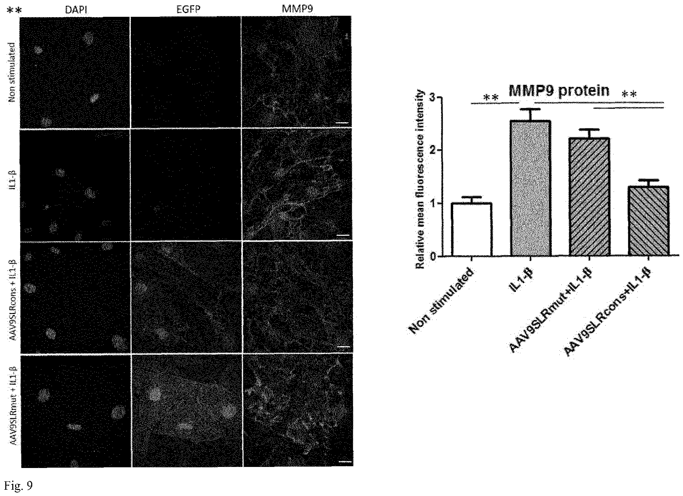

Figure 9: AAV9SLR-mediated expression of the hpAP-1 consensus RNA decoy ODN significantly decreases MMP-9 protein abundance in vascular SMCs. MMP-9 (red) was detected by immunofluorescence analysis. Successful transduction was confirmed by eGFP fluorescence (green). The graph summarizes quantification of the mean fluorescence intensity in the different treatment groups. Nuclei were visualized by DAPI staining (blue). The scale bar represents 25 µm *(n=4,* 20 images were analysed per group and individual experiment, **p<0.01).

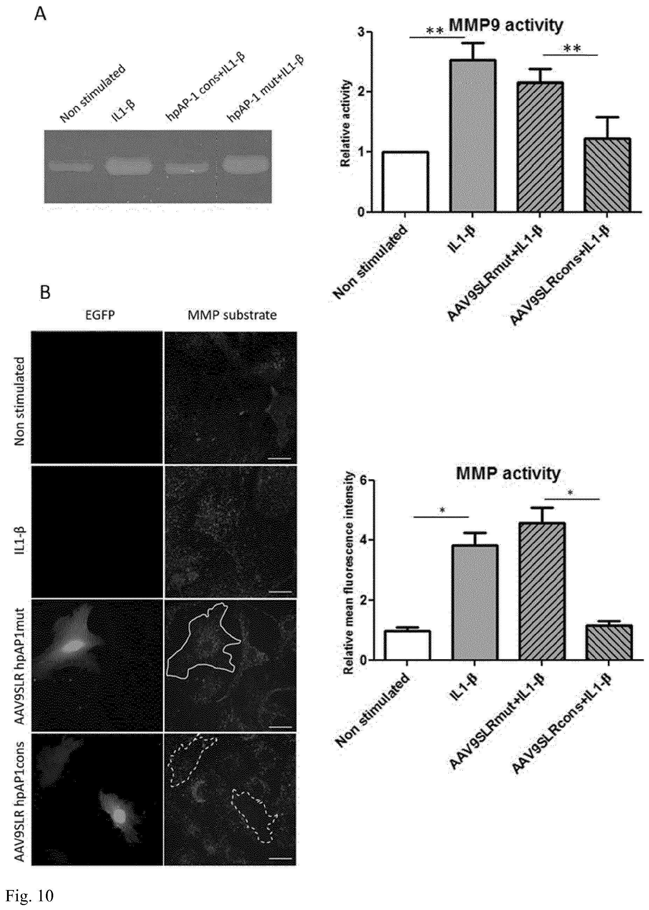

Figure 10: AAV9SLR-mediated expression of the hpAP-1 consensus RNA decoy ODN significantly decreases MMP activity in vascular SMCs. (A) Gelatinolytic activity of MMP-9, detected as 105 kDa white bands on Coomassie Brilliant Blue stained zymography gel. The graph shows quantification of the relative densitometric analysis of the bands obtained in the different treatment groups. Values were normalized to the non-stimulated control group (*n*=4, *p<0.05). (B) Representative images of SMCs treated with MMP substrate after AAV9SLR transduction and IL-1β stimulation. The degree of red fluorescence is proportional to the amount of cleaved MMP substrate. Transduced eGFP positive cells (green, underlined with white) were selected for quantitative analysis. Scale bar represents 25 µm *(n=4,* 20 images were analysed per group and individual experiment, **p<0.01).

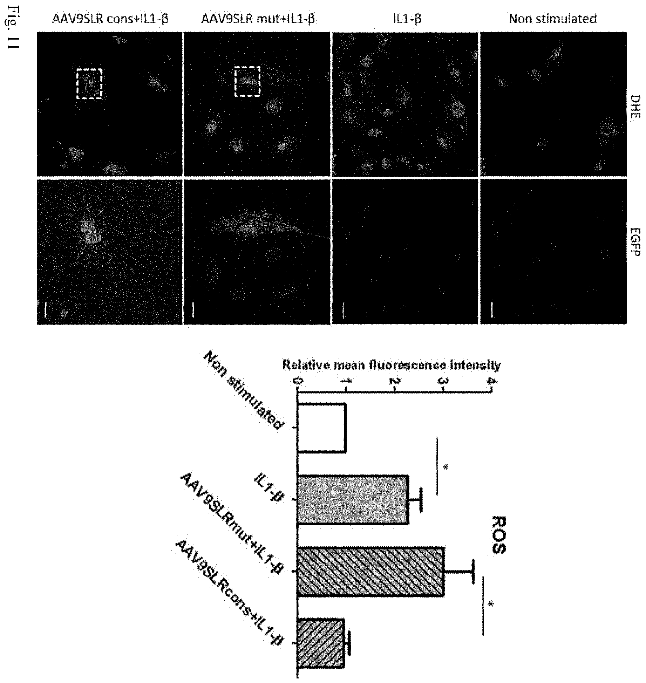

Figure 11: AAV9SLR-mediated expression of the hpAP-1 consensus RNA decoy ODN decreases the amount of ROS in vascular SMCs. (Left) Representative images showing the measurement of ROS (DHE-based) in mgR/mgR SMCs following AAV9SLR transduction and exposure to IL-1β. The graph summarizes quantification of the relative mean fluorescence intensity levels as compared to the non-stimulated control. EGFP fluorescence (green) shows successfully transduced cells. Scale bar represents 25 µm *(n=4,* 20 images were analysed per group and individual experiment, *p<0.05).

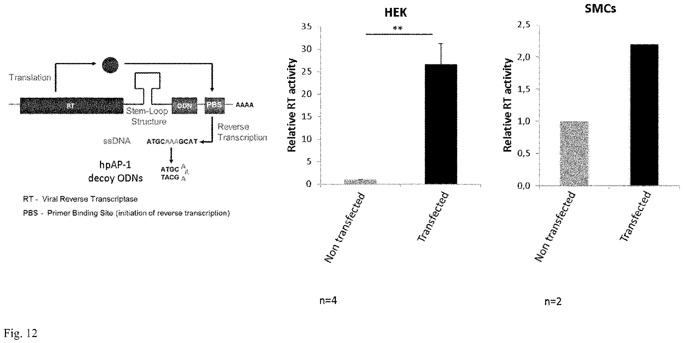

Figure 12: (Right) Principle of the expression of a hpAP-1 consensus cDNA decoy ODN in the target cell and (right) successful transfection of HEK and vascular SMCs with the plasmid-based expression construct as determined by monitoring RT activity with the C-Type Reverse Transcriptase Activity Kit (Cavidi, Sweden; *n*=2-4, *p<0.05).

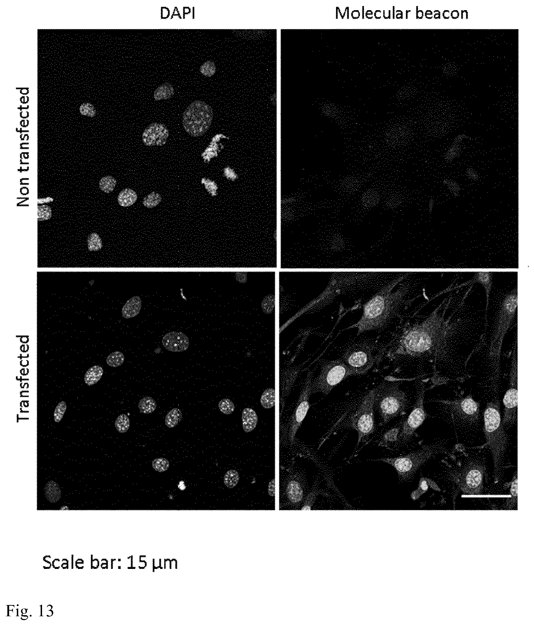

Figure 13: Verification of the expression of the hpAP-1 consensus cDNA decoy ODN after transfection of vascular SMCs by using the molecular beacon technique. Shown are representative fluorescence microscopy images, the scale bar corresponds to 15 µm.

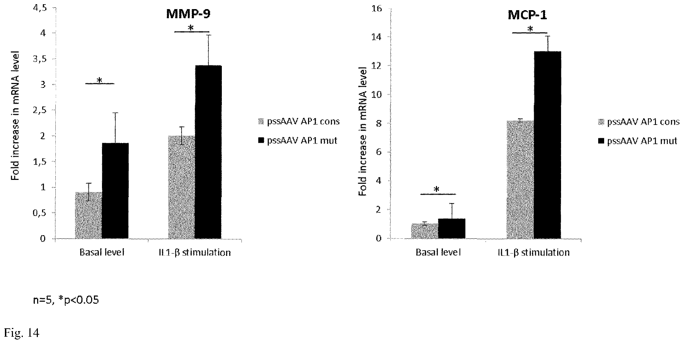

Figure 14: Plasmid-mediated hpAP-1 consensus cDNA decoy ODN expression in vascular SMCs significantly decreases IL-1β induced MMP-9 and MCP-1 mRNA expression (*n*=5, *p<0.05).

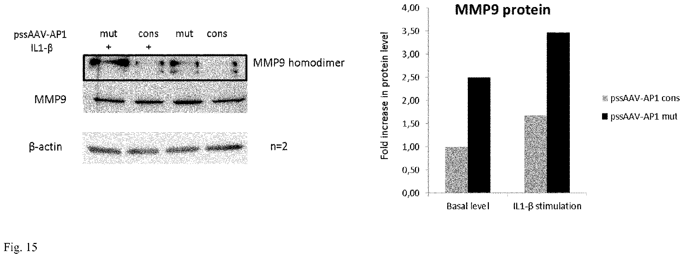

Figure 15: Plasmid-mediated hpAP-1 consensus cDNA decoy ODN expression in vascular SMCs decreases basal and IL-1β induced MMP-9 protein abundance (*n*=2, exemplary experiments).

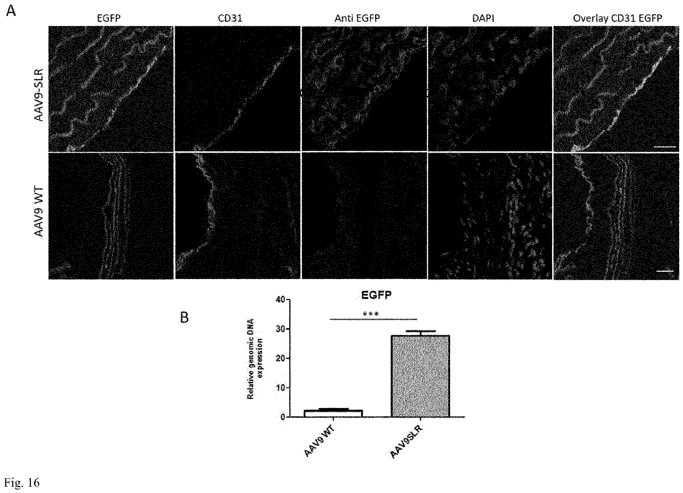

Figure 16: Capsid peptide SLR significantly enhances *ex vivo* transduction efficiency of AAV9 in aortic grafts of mgR/mgR mice. (A) (Immuno)fluorescence analysis showing eGFP expression (green) in CD31-positive endothelial cells (red) as well as in vascular SMCs (magenta) in the media. (B) EGFP expression on the genomic DNA level is increased in AAV9SLR transduced grafts as compared to AAV9WT treated tissue, 30 days after transduction and reimplantation. Scale bar represents 25 µm *(n=4,* ***p<0.001).

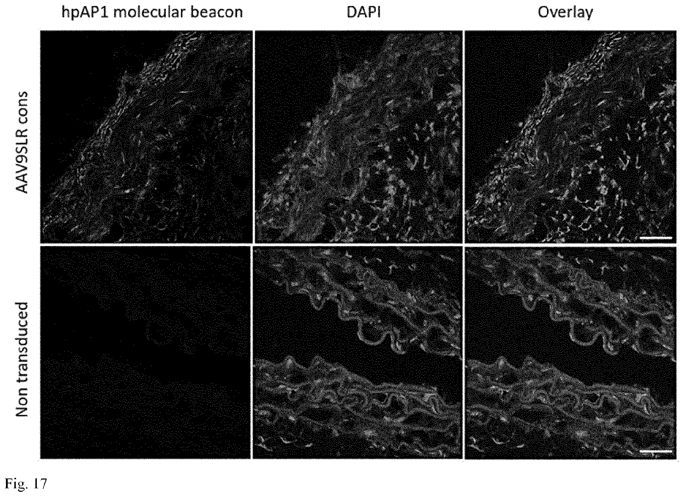

Figure 17: Verification by using the molecular beacon technique (red fluorescence) that the hpAP-1 consensus RNA decoy ODNs is expressed in the aortic vessel wall after ex *vivo* pretreatment of mgR/mgR aortic grafts 30 days post implantation. DAPI staining (blue) was used to visualize the nuclei. Decoy ODN related fluorescence is detected both in the nucleus and in the cytoplasm of the transduced cells. The scale bar represents 25 µm (*n*=4, exemplary images).

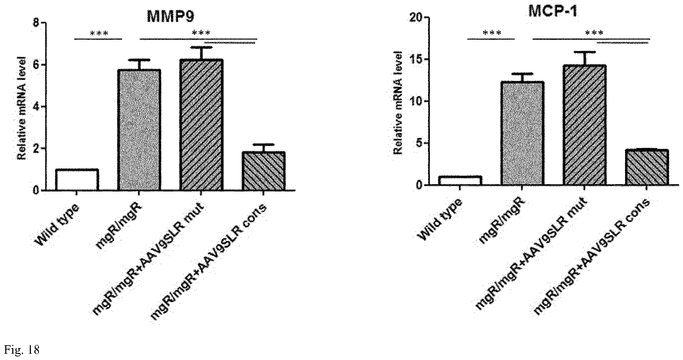

Figure 18: AAV9SLR-mediated expression of the hpAP-1 consensus RNA decoy ODN significantly decreases AP-1 target gene expression in *ex vivo* transduced mgR/mgR aortic grafts. MMP-9 and MCP-1 mRNA levels were normalised to RPL32 as housekeeping gene and expressed relative to the mRNA level in aortic grafts isolated from age-matched wild type mice (*n*=6, ***p < 0.001).

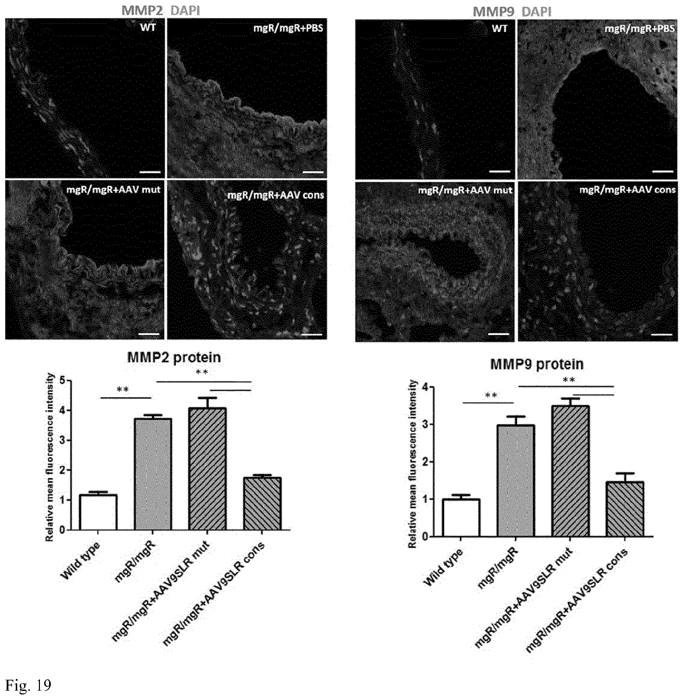

Figure 19: AAV9SLR-mediated expression of the hpAP-1 consensus RNA decoy ODN significantly decreases MMP-2 and MMP-9 protein abundance in *ex vivo* transduced mgR/mgR aortic grafts. (Top) Representative images showing MMP-2 and MMP-9 specific immunofluorescence (red). DAPI staining (blue) was used to mark nuclei. (Bottom) Relative mean fluorescence intensity was analysed and normalized to the value of the wild type control mice. The scale bar represents 25 µm (n=6, 20 images were analysed per group and mouse, **p<0.01).

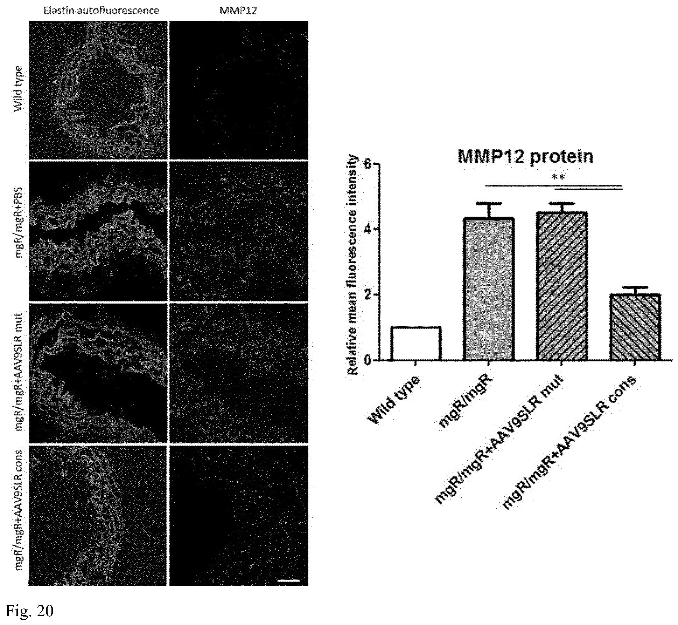

Figure 20: AAV9SLR-mediated expression of the hpAP-1 consensus RNA decoy ODN significantly decreases MMP-12 protein abundance in *ex vivo* transduced grafts. (Left) Representative immunofluorescence images showing MMP-12 (magenta) staining in cryosections of the aortic grafts. (Right) Quantification of the mean fluorescence intensity, normalized to the level of MMP-12 specific immunofluorescence in control mice. Elastin autofluorescence was recorded in the green channel. Scale bar represents 50 µm (n=6, 20 images were analysed per group and mouse, **p<0.01).

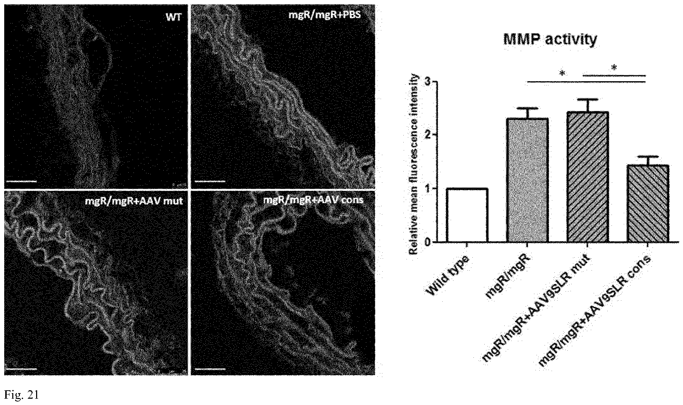

Figure 21: AAV9SLR-mediated expression of the hpAP-1 consensus RNA decoy ODN significantly decreases MMP activity in *ex vivo* transduced mgR/mgR aortic grafts. (Left) Representative images depicting the fluorescence intensity (red) of the cleaved MMP substrate in aortic cryosections. Elastin fibers were detected by imaging autofluorescence in the green channel. Nuclei were detected by DAPI staining (blue). (Right) Quantification of relative mean red fluorescence intensity in the different treatment groups. MMP activity was normalized to the value detected in aortic cryosections of wild type control mice. Scale bar represents 25 µm (*n*=6, 20 images were analysed per group and mouse, *p<0.05).

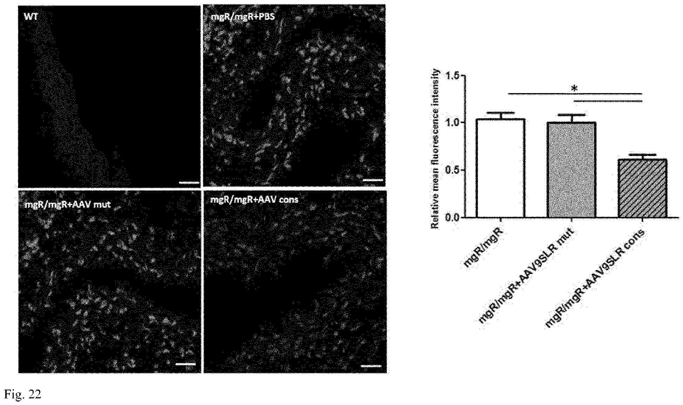

Figure 22: AAV9SLR-mediated expression of the hpAP-1 consensus RNA decoy ODN significantly diminishes the amount of ROS generated in *ex vivo* transduced mgR/mgR aortic grafts. DHE fluorescence (red) was quantified in the different treatment groups and normalized to the mean fluorescence intensity derived from conversion of DHE to ethidium bromide in non-treated mgR/mgR aortic grafts. (Left) Representative images, (right) statistical summary of the data. The scale bar represents 25 µm (*n*=6 for mgR/mgR mice, 20 images were analysed per group and mouse, *p<0.05).

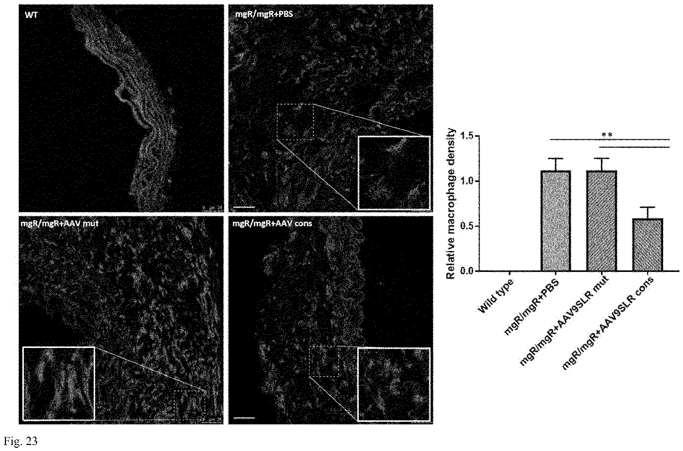

Figure 23: AAV9SLR-mediated expression of the hpAP-1 RNA decoy ODN significantly decreases monocyte/macrophage infiltration in the adventitia of the *ex vivo* transduced aortic grafts. (Left) Representative images showing F4/80 immunofluorescence (red) used to mark the macrophages. Elastin autofluorescence was recorded in the green channel and DAPI (blue) was used to mark the nuclei. (Right) Quantification of relative macrophage density in the adventitia normalized to the level in cryosections of untreated wild type control mice. The scale bar represents 50 µm (*n*=6, 20 images were analysed per group and mouse, **p<0.01).

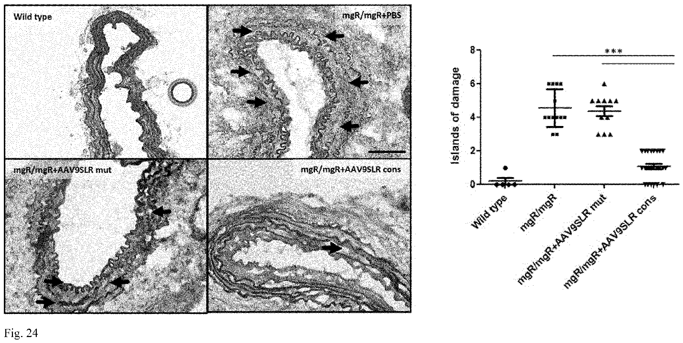

Figure 24: AAV9SLR-mediated expression of the hpAP-1 consensus RNA decoy ODN protects the elastic fiber structure in *ex vivo* transduced aortic grafts from mgR/mgR mice. (Left) Representative images of elastic van Gieson staining in the four experimental groups and (right) quantification of the frequency of islands of damage per field of view, indicated on the left by the black arrows. The scale bar represents 50 µm (*n*=6, 20 images were analysed per group and mouse, ***p<0.001).

The following examples shall merely illustrate the invention. They shall not be construed, whatsoever, to limit the scope of the invention.

### "Naked" hpAP-1 consensus decoy ODN effects on mgR/mgR SMCs expression of AP-1 target genes

In order to activate the AP-1 transcription factor family in the cultured vascular smooth muscle cells (SMCs), interleukin-1β (IL-1β) was applied to serum depleted cells isolated from the aorta of 3 months old mgR/mgR mice. Eight hour exposure to IL-1β caused a significant increase in the expression of both MMP-9 and MCP-1, whereas pre-treatment with the hpAP-1 consensus decoy oligodeoxynucleotides (ODNs) for 2 hours led to a significant reduction in mRNA abundance of both MMP-9 and MCP-1. As shown in Figure 1, the control hpAP-1 mut decoy ODNs had no such effect.

### MMP expression and activity in vascular SMCs derived from mgR/mgR mice

Next, the effect of the hpAP-1 consensus decoy ODN on MMP-9 protein abundance in the mgR/mgR vascular SMCs was investigated. As shown in Figure 2, MMP-9 protein in these cells was mainly detected as a homodimer, the abundance of which was reduced by 74% upon pretreatment of the vascular SMCs with the hpAP-1 consensus but not mutant control ODN. Also MMP-9 specific as well as total MMP activity were significantly decreased after prior exposure of the vascular SMCs to the consensus decoy ODN (Fig. 3). The mutant control ODN had no such effect.

### Generation of reactive oxygen species (ROS)

The generation of ROS in the aortic vessel wall of patients with Marfan syndrome may play an important role in aortic aneurysm formation and rupture, and may be exacerbated through TGF-β signaling. Therefore, the effects of the hpAP-1 consensus and mutant control ODN on ROS formation in the mgR/mgR vascular SMCs were investigated. As shown in Figure 4, ROS formation significantly increased in IL-1β treated SMCs, and this effect was significantly inhibited by pretreatment of the SMCs with the consensus but not the mutant control ODN.

### Vascular SMC migration

One hallmark of the switch of the phenotype of vascular SMCs during remodeling processes from the quiescent contractile to the activated synthetic state is an enhanced migration capacity that goes hand in hand with an increased SMC-derived MMP-mediated degradation of the extracellular matrix. Eventually, when appropriately stimulated, vascular SMCs migrate from the tunica media to the tunica intima during most remodeling processes. As shown in Figure 5, using a 2D lateral migration assay, IL-1β significantly enhanced migration of the vascular SMCs, and this migration was essentially abolished following pretreatment with the hpAP-1 consensus but not mutant control ODN.

### Transduction efficacy of different unmodified and modified adenoassociated viral vectors

To analyse which viral vector most effectively transduces the murine vascular smooth muscle cells, 4 modified AAV2, 3 modified AAV9 and one unmodified (wild type) AAV9 carrying a construct encoding for enhanced green fluorescent protein (eGFP) were administered to the vascular SMCs at the same multiplicity of infection (MOI). Three days post transduction, eGFP-dependent fluorescence was monitored to assess the percentage of successfully transduced cells. As shown in Figure 6, the AAV9 harbouring the targeting peptide SLRSPP (AAV9SLR) was by far the most efficient adenoassociated viral vector transducing more than 70% of the vascular SMCs within 3 days.

### AAV9SLR-mediated expression of short hairpin RNA-based decoy ODNs in vascular SMCs

Next the SMCs were transduced with AAV9SLR vectors harbouring the eGFP expression construct only or harbouring a construct that in addition to eGFP gives rise to expression of a 44 nucleotide RNA-based hpAP-1 consensus decoy ODN (SEQ ID NO:10). The molecular beacon technique (see below) in combination with fluorescence microscopy demonstrated successful expression of the consensus decoy ODN in the cultured vascular SMCs following transduction with the AAV9SLR (Fig. 7).

Vascular SMCs thus transduced revealed a dramatic decline in IL-1β-mediated mRNA expression of the prototypic AP-1 target genes *MMP9* and *CCL2* (MCP-1) while expression of the RNA-based mutant control ODN essentially had no effect (Fig. 8). Similarly, MMP-9 protein abundance (Fig. 9), MMP-9 specific activity and overall MMP activity (Fig. 10) were all markedly reduced upon expression of the consensus decoy ODN but not the mutant control ODN in these cells. The same held true for IL-1β-stimulated ROS formation in the vascular SMCs (Fig. 11).

### Plasmid-mediated transfection of vascular SMCs with a hp-AP-1 consensus DNA-based decoy ODN expressing construct

In addition, the possibility was explored to express a cDNA-based hpAP-1 decoy ODN in vascular SMCs and test its biological activity. To this end, an expression vector was designed encoding a viral reverse transcriptase (RT) and the corresponding ODN flanked by a primer binding site. Both sequences are separated by a stem loop structure so that the RT is first transcribed and translated into the active enzyme which then reverse transcribes the ODN resulting in the formation of a single stranded cDNA molecule. The cDNA hybridizes with itself only leaving the hairpin single stranded. The principle of this approach is depicted in Figure 12 (left panel).

Subsequently, the expression vector was cloned into a plasmid which was then transfected into HEK cells or vascular SMCs. Successful transfection was verified by the resulting increase in RT activity over baseline in the transfected cells (Fig. 12, right panel). In addition, the molecular beacon technique was used to verify expression of the cDNA-based hpAP-1 consensus decoy ODN by the vascular SMCs 2 days post transfection (Fig. 13). As shown in Figure 14, plasmid-mediated delivery of the cDNA-based hpAP-1 consensus decoy ODN to the vascular SMCs significantly inhibited IL-1β-stimulated MMP-9 and MCP-1 mRNA expression as compared to transfection with the mutant control ODN expressing plasmid. The same held true for MMP-9 protein abundance in these cells (Fig. 15).

### Ex vivo transduction of aortic grafts from mgR/mgR mice with the AAV9SLR vector harbouring the expression construct for the hpAP-1 consensus RNA-based decoy ODN

First, it was tested whether *ex vivo* treatment of the aortic grafts derived from mgR/mgR mice with the AAV9SLR-eGFP expressing vector followed by infrarenal implantation of these grafts into mgR/mgR recipient mice at 30 days post implantation had resulted in a significant upregulation of eGFP expression in the vessel wall. As compared to AAV9WT-eGFP transduced aortic grafts, there was a striking increase in eGFP detection in the AAV9SLR-eGFP transduced aortic grafts (Fig. 16), confirming that this adenoassociated viral vector is especially suited to effectively transduce the aortic vessel wall upon topical or local application.

Next, by employing the molecular beacon technique and DAPI counterstaining of nuclei, expression of the hpAP-1 consensus RNA-based decoy ODN was verified in transplanted aortic grafts that had been transduced with the corresponding adenoassociated viral vector (Fig. 17). Aortic grafts transduced the same way revealed a dramatic decline in MMP-9 and MCP-1 mRNA expression in the aortic vessel wall 30 days post implantation while grafts transduced with the mutant control ODN expressing AAV9SLR did not differ from vehicle-treated mgR/mgR grafts (Fig. 18). Likewise, MMP-2, MMP-9 (Fig. 19) and MMP-12 protein abundance (Fig. 20) in the implanted grafts was markedly reduced following *ex vivo* pre-treatment with the hpAP-1 consensus RNA-based decoy ODN expressing AAV9SLR. The same held true for total MMP activity (Fig. 21), ROS formation within the vessel wall of the aortic graft (Fig. 22) and infiltration of monocyte/macrophages (Fig. 23). Most importantly, elastolysis determined by the number of islands of damage (strand breaks) within the elastin fibers of the vessel wall of the differently pretreated mgR/mgR aortic grafts was dramatically reduced following transduction with the AAV9SLR expressing the hpAP-1 consensus decoy ODN as compared to the two control groups (Fig. 24).

The following section describes the methods used to conduct the *afore-mentioned in vitro* and *in vivo* studies.

### Isolation and culture of primary SMCs

For the isolation of primary murine SMCs, the thoracic aorta of fibrillin-1 deficient mice (mgR/mgR) was used. Mice were sacrificed by CO₂ inhalation, the chest was cleaned with 70% ethanol before opening and the heart was exposed. Next, 5 mL of cold sterile NaCl solution (Braun, Melsungen, Germany) was perfused through the left ventricle using a 23 gauge needle syringe in order to remove blood present in the aorta. The thoracic aorta was isolated, cleared from fat and connective tissue and cut into small pieces under sterile conditions in complete DMEM medium. The tissue pieces were incubated at 37°C, 5% CO₂ for 24 hours in the presence of 100 µmol/L collagenase I (Worthington Biochemical Corporation, Lakewood, USA). Afterwards, medium was replaced and explants were further cultured until cells reached 80% confluency. Lastly, cells were passaged and either propagated, frozen in liquid nitrogen or used for experiments up to passage 5, in order to avoid the phenotypical changes that occur with long-term subculture of vascular SMCs. Cells were cultured either on plastic, for qPCR and Western blot experiments, or on gelatine-coated glass coverslips, when the assays demanded the use of confocal microscopy.

### In vitro naked hairpin decoy ODN treatment and transduction with AAVs

Vascular SMCs were seeded in culture dishes the day before treatment and allowed to adhere overnight. Hairpin decoy ODNs were added in cell culture medium to a final concentration of 10 µmol/L and incubated for 2 hours before stimulation of the cells.

AAV transduction was conducted at a MOI of 10⁵ viral particles/cell in serum-free medium. After the 'infection period' (12-16 hours), the culture medium was replaced. Cells were monitored by fluorescence microscopy for eGFP expression 3 days after transduction.

Vascular SMCs were treated with a series of AAV serotypes with targeting capsides (AAV2NDV, AAV2APV, AAV2DLG, AAV9RGD, AAV9SLR, AAV9WT, provided by Dr. Karl Varadi, Clinic for Cardiology, Angiology and Pneumology, University Clinics Heidelberg) expressing eGFP to detect expression of the viral constructs and determine transduction efficacy. Fluorescence was visualized after 3 days using a fluorescence microscope.

In order to activate AP-1, vascular SMCs were serum-depleted overnight and IL1-β (R&D Systems, Wiesbaden, Germany) was supplemented to a final concentration of 20 ng/mL for 24 hours for monitoring changes in protein abundance or 8 hours for examination of target gene mRNA expression.

### Assessment of SMC 2D migration

Vascular SMCs were cultured until confluency in migration inserts (Ibidi, Munich, Germany) followed by pretreatment with naked hairpin decoy ODNs or transduction with adenoassociated viral vectors. Afterwards, IL-1β (20 ng/mL) was added to the medium and inserts were removed to create an empty space in between the cells. Images were taken at various time points between 0-24 hours with a brightfield microscope (4x magnification) and the area between cells was analysed using ImageJ. Relative migration capacity was calculated as follows: percent migration = (initial wound area - final wound area)/initial wound area × 100.

### AAV production

AAV production was performed in collaboration with Prof. Oliver Müller, Dr. Andreas Jungmann, Clinic for Cardiology, Angiology and Pneumology, University Clinic Heidelberg according to standard protocols (Varadi et al. (2012), Gene Therapy 19:800).

### Cloning of hpRNA decoy ODN expressing plasmids

The hpRNA decoy ODNs were generated as shRNAs under the H1 promoter (plasmid backbone provided by Prof. Oliver Müller and Dr. Andreas Jungmann. The plasmids encoded eGFP as an expression marker under the control of a CMV promoter, and included inverted terminal repeats (ITR), which are crucial for AAV production. The individual sequences for each shRNA subcloning were ordered as gene synthesis (see table below). The synthetized gene sequences contained part of the H1 promoter (5'-end) and the shRNA (3'-end) flanked by KasI and XhoI restriction sites.

**Table List of gene synthesis sequences. Sequences recognized by restriction enzymes are presented in italic and decoy ODN sequences are shown in bold.**

| Plasmid | Sequence |
|---|---|
| P-AP-1 cons (SEQ ID NO:11) | 5'-AGGCGCCCTGCAATATTTGCATGTCGCTATGTGTTCTG GGAAATCACCATAAACGTGAAATGTCTTTGGATTTGGGA ATCTTATAAGTTCTGTATGAGACCACAGTCGAC CTGCGGTGCTGACTCAGCACGAAACGTGCTGAGTCAGCACCGCAG CACCGCAGTTTCGACCTCGAGA-3' |
| P-AP-1 mut (SEQ ID NO:12) | 5'-AGGCGCCCTGCAATATTTGCATGTCGCTATGTGTTCTG GGAAATCACCATAAACGTGAAATGTCTTTGGATTTGGGA ATCTTATAAGTTCTGTATGAGACCACAGTCGAC CTGCGGTGCTTACTTAGCACGAAACGTGCTAAGTAAGCACCGCAG CACCGCAGTTTCGACCTCGAGA-3' |

### Maintainence of bacterial cells

For AAV plasmid cloning, recombination-deficient SURE2 (Stop Unwanted Rearrangement Events 2) bacterial cells (Agilent Genomics, Waldbronn, Germany) were used, in order to prevent the deletion of ITR sequences. Liquid cultures, grown in sterile LB medium were expanded at 37°C under continuous agitation (150-200 rpm) in a bacterial incubator. Bacteria stocks for transformation were stored as 50 µL aliquotes at -80 °C.

### Digestion of plasmid DNA

Plasmid digestion with KasI and XhoI restriction enzymes was performed in the corresponding buffers for 2 h for each µg plasmid DNA at 37°C. Reaction efficiency was analyszed by agarose gel electrophoresis (100 V for approximately 70 min). A molecular weight ladder was used to determine band size (GeneRuler 1 kb DNA Ladder, Thermo Fischer Scientific, Munich, Germany). The DNA was imaged by using a GelDoc XR unit and analyzed using the Quantity One software package version 4.06 (Bio Rad, Munich, Germany). The DNA bands corresponding to the backbone and the insert were cut out and DNA was purified from the gel using QIAquick Gel Extraction Kit (Qiagen, Hilden, Germany) according to the manufacturer's instructions. Eluted DNA concentration was measured using NanoDrop spectrophotometer (PeQlab Biotechnologie, Erlangen, Germany).

### Modification of DNA ends

In order to reduce the process of backbone re-ligation, 5'-phosphate ends were removed by using Antarctic phosphatase (New England Biolabs, Frankfurt am Main, Germany). The gel-purified vector backbone was incubated with the enzyme in the specific buffer for 30 min at 37°C. Phosphatase heat-inactivation was afterwards performed at 65°C for 30 min on a thermoblock (Eppendorf, Hamburg, Germany).

### Ligation of plasmid DNA

Ligation of DNA fragments was performed in a ratio of 1:4 backbone:insert by using T4 DNA ligase in the specific ATP-containing buffer provided by the producer. For each ligation reaction, 50 µg backbone was used. The dephosphorylated backbone was incubated with the insert in the presence of ligase for 16 hours at 16°C using a thermoblock. The reaction mixture was further used for transformation of the bacteria.

### Bacterial transformation

Fifty µL of bacteria were allowed to thaw on ice and then incubated with the ligation mixture for 30 min at 4°C. Heat-shock was performed at 42°C for 45 s, on a thermoblock. Afterwards, bacteria were transferred on ice for 10 min and then cultured for 30 min at 37°C in 300 µL LB medium without antibiotics. Next, the suspension was spread on sterile LB-plates with 15% agar and 100 µg/mL ampicillin (Sigma-Aldrich, Munich, Germany). Plates were incubated overnight at 37°C to allow colony formation. Individual bacterial colonies were randomly picked and further cultured in 5 mL LB medium with ampicillin for 16 hours. Plasmid DNA was isolated using the QIAprep Spin Miniprep Kit (Qiagen, Hilden, Germany) according to the manufacturer's instructions.

The presence of the insert was confirmed by KasI and XhoI digestion as well as DNA sequencing (Eurofins Genomic, Luxembourg). Positive colonies were expanded in 1 L of ampicillin containing LB medium overnight and DNA was isolated using the ZymoPURE Plasmid Gigaprep Kit (Zymo Research, Freiburg, Germany) according to the manufacturer's instructions. Each preparation yielded at least 1.5 g plasmid DNA, which was further used for AAV preparation.

### Quantitative real time PCR

Total RNA was extracted from cells or aortic tissue using the RNeasy Mini Kit (Qiagen, Hilden, Germany) following the manufacturer's instructions and RNA concentration was measured using the NanoDrop spectrophotometer. First strand synthesis of cDNA was completed using Omniscript Reverse Transcriptase kit (Qiagen) and OligodT primers (Promega, Mannheim, Germany), starting from equal amounts of RNA for each sample. SYBR Green (Qiagen) qRT-PCR was performed using the Qiagen Rotor-Gene cycler in reactions of 20 µL (10 µL SYBR Green, 2 µL primer mix, 3 µL RNase free water, 5 µL cDNA). cDNA was amplified using specific primers for the genes of interest. The qPCR program consisted of an initial denaturation step of at 95°C for 5 min followed by 40 cycles of denaturation (at 95°C for 60 s), annealing (primer dependent temperature, 25 s) and elongation (72°C for 60 s) followed by monitoring of the melting curve. The presence of a single amplicon was determined by the existence of a single peak in the melting curve graph. Data was analyzed by relative quantification using the double delta Ct (cycle threshold) method with RPL32 as a housekeeping gene (Pfaff MW (2001) A new mathematical model for relative quantification in real-time RT PCR. Nucleic Acids Res 29: 2003-2007). The Ct was defined as the cycle number at which the fluorescence signal crosses the fluorescence threshold, considered as background. Corresponding controls (samples isolated from control mice) were used for relative gene expression calculation.

### In situ hybridization

For the detection of RNA decoy ODNs *ex vivo,* samples (tissue sections) were subjected to RNA in situ hybridization. In brief, 5-µm tissue cryosections were fixed with DEPC (diethyl dicarbonate)-treated 4% p-formaldehyde (Sigma-Aldrich, Munich, Germany) for 10 min followed by incubation with 20 µg/mL proteinase K (Sigma-Aldrich, Munich, Germany) at 37°C for 15 min in order to induce tissue permeabilization and antigen retrieval. As a probe, a molecular beacon (Biomers, Ulm) with complementary sequence to the hairpin decoy ODNs with 5'-Cy5 labeling and 3'-BHQ-2 (Black Hole Quencher) as a quencher was used. In the hybridized state, the molecular beacon itself does not emit a fluorescence signal due to the dye and the quencher being located proximal to each other. After hybridization to the target sequence, red fluorescence can be detected (Wile et al. (2014), Nature Protocols 9:2411). Prior to hybridization, the molecular beacon was heated to 95°C followed by gradual cool-down to ambient temperature to allow the hairpin structure to form; afterwards it was kept on ice until use.

Samples were incubated overnight at 55 °C with the probe dissolved in hybridization buffer in a humidified atmosphere. As blocking reagents, salmon DNA and yeast tRNA were added to the mixture. After extensive washes (3 × 20 min) in hybridization buffer and DEPC-treated PBS (1 × 15 min) to remove non-hybridized molecular beacon, EeGFP immunofluorescence staining was performed. Finally, DAPI staining was performed (excitation at 358 nm/emission at 461 nm) for visualization of the nuclei using Fluoroshield with DAPI mounting medium (Sigma-Aldrich).

### Protein extraction and Western blot analysis

Total protein was extracted from SMCs using complete RIPA buffer and quantified using Bradford method (Bio Rad, Munich, Germany). The lysate was snap-frozen and stored at -80°C until further use. For protein analysis, 30 µg of protein were denatured by incubation at 95°C for 10 min with 4 × sample buffer (Roth, Karlsruhe, Germany) and loaded onto 12% polyacrylamide gels with SDS. A pre-stained protein standard (Bio Rad, Munich, Germany) was used for molecular weight estimation. Separated proteins were transferred onto a methanol pre-activated PVDF (polyvinylidene difluoride) membrane (Merck Millipore, Darmstadt, Germany) with a pore size of 0.45 µm. Ponceau S (Roth) staining was used to document equal loading of protein samples. The membranes were then digitized using a Lexmark scanner. Prior to further processing, the membranes were completely destained by washing with TBS.

Blocking was made using 5% nonfat dry milk diluted in TBS-T for one hour, followed by overnight incubation at 4°C under continuous shaking with the primary antibodies; β-actin was used as a loading control. After washing, membranes were incubated with the corresponding horseradish-peroxidase labeled secondary antibodies for 1 hour at ambient temperature. Membranes were developed with the chemoluminescent substrates Luminata Classico (Merck, Darmstadt) for β-actin and Luminata Forte (Merck) for MMP-9 detection for 3 min. Afterwards, membranes were imaged using the ImageQuant LAS 4000 mini system (GE Healthcare Life Sciences, Munich, Germany). The band intensities were analyzed and related to β-actin using ImageJ software.

### Immunofluorescence analysis

For immunocytochemistry experiments, vascular SMCs were plated on glass coverslips pre-coated with gelatine. After treatments, cells were washed with PBS and fixed with 4% PFA for 5 min. Subsequently, the glass slides were washed and blocked in 3% BSA (Sigma-Aldrich) and 0.05% Triton-X100 as a permeabilization reagent, dissolved in PBS. Primary antibodies were diluted in blocking buffer and incubated overnight at 4°C. Next, a set of 3 washing steps with PBS was performed, followed by fluorescently labelled secondary antibody incubation for 1 hour at ambient temperature and DAPI nuclear staining. Images were taken using confocal microscopy (Leica TCS SP8, Leica Microsystems, Mannheim) and mean fluorescence intensity was calculated using ImageJ.

Similarly, aortic tissue was collected and washed in NaCl to remove blood residuals. Next, the tissue was embedded into TissueTek (Leica Biosystems, Wetzlar, Germany) and snap-frozen by immersion into liquid nitrogen. A cryotome (Leica) was used to section the frozen tissue (temperature -21°C) into 5-µm thick cryosections that were fixed with Zinc fixative and blocked with PBS containing 3% BSA and 0.05% Triton-X100 for 1 hour. Primary antibodies against the proteins of interest were diluted in blocking buffer and incubated overnight at 4°C in a humidified atmosphere. As secondary antibodies, compatible Cy3 or Cy5 labeled IgGs were used, diluted in blocking buffer. Nuclei were visualized by DAPI counterstaining and images were recorded by confocal microscopy (Leica TCS SP8). Relative mean fluorescence intensity was measured using ImageJ as previously described (Jensen (2013), The Anatomical Record 296:378) and related to the corresponding controls.

### Gel zymography

Gel zymography was used for the detection of MMP activity secreted by vascular SMCs. Cell culture supernatant was incubated for 24 hour on a rotor at 4°C with gelatin-coated beads (Sepharose 4B Beads, Sigma Aldrich, Munich, Germany) in order to concentrate the MMPs present in the medium. Afterwards, MMPs were eluted using non-reducing sample buffer followed by electrophoretic separation on 5 mg/mL gelatine-containing acrylamide gels. A standard ladder was used to identify the molecular weights of the bands. Following electrophoresis, gels were incubated for 2 hour in washing buffer containing Triton X-100 to remove the SDS and renature the enzymes. Afterwards, gels were placed at 37°C in an incubator overnight in incubation buffer containing CaCl₂, which is essential for enzymatic activity of MMPs. Gels were then transferred to Coomassie Brilliant Blue staining solution for 1 hour at room tempera-ture under shaking and afterwards incubated in destaining buffer until clear bands were visible on the blue background. These bands corresponded to the level of gelatinase activity, and were further quantified. For this purpose, gels were scanned and the relative band intensity was measured using ImageJ.

### In situ zymography

*In situ* zymography was performed using a broad spectrum MMP substrate (520 MMP FRET Substrate XIV, Anaspec, Göttingen, Germany), which detects the activity of MMP-1, 2, 3, 7, 8, 9, 12, and 13 present in the sample. For *in vitro* measurement of MMP activity, vascular SMCs were treated with the substrate to a final concentration of 0.1 µg/mL in serum-free medium for 1 hour at 37°C. After cell fixation with 4% PFA and DAPI staining, the cells were visualised using a confocal microscope and red fluorescence (Ex/Em=494/521 nm) was analysed as a measure of MMP activity.

Similarly, MMP activity was analysed in 5 µm thick aortic cryosections. The tissue was fixed with Zinc fixative and incubated with the substrate at 37°C for 4 hours in a humidified atmosphere to avoid evaporation of the substrate solution. The sections were then extensively washed to remove the non-bound substrate. Red fluorescence intensity was measured and calculated relative to the level in wild type control aortic cryosections.

### Determination of ROS formation

The level of oxidative stress *in vitro* was measured using 2',7'-dichlorofluorescin diacetate (DCFDA, Sigma Aldrich) or, when vascular SMCs were transduced with eGFP expressing AAVs, with dihydroethidium (DHE, Sigma Aldrich). For this purpose, cells were cultured on gelatine-coated glass coverslips and subjected to decoy ODN treatement or transduction with AAV9SLR. Afterwards, cells were incubated with DCFDA (5 µmol/L) or DHE (0.1 µmol/L) for 30 min at 37°C in serum-free medium. The dye was then removed by washing with PBS and cells were stimulated with IL1-β for 6 hours. Non-stimulated cells were used as control. Next, coverslips were washed again with PBS and fixation was performed using methanol. After DAPI nuclear staining, cells were visualised using a confocal microscope (DCFDA: Ex/Em=485/535nm; DHE: Ex/Em=518/605) and the fluorescence intensity was measured using ImageJ.

Aortic cryosections were fixed with 4% PFA for 5 min and rinsed in PBS. DHE was applied to the tissue to a final concentration of 10 µmol/L and incubated at 37°C for 30 min in a humidified atmosphere. The sections were then briefly washed with PBS and visualised by confocal microscopy. Mean fluorescence intensity was measured using ImageJ.

### Verhoeff-van Gieson staining

Elastic van Gieson staining was performed using the Elastic Stain Kit (Sigma Aldrich) according to the manufacturer's instructions. In brief, 5 µm cryosections were fixed with 4% PFA (paraformaldehyde) and allowed to dry at room temperature. Afterwards, sections were stained with working elastin solution (20 mL hematoxylin solution, 3 mL FeCl₃, 8 mL Weigerts iodine solution, 5 mL deionized H₂O) for 10 min, and briefly washed with deionized H₂O. Aortic tissue was then differentiated in 3% FeCl3 for 1 min, rinsed in 95% ethanol and stained with van Gieson solution for 1 min. The sections were washed with 95% ethanol and mounted. Images were taken using brightfield microscopy with 10× magnification. Elastin fragmentation was assessed by counting the number of islands of damage by two researchers blinded to the treat-ment. An island of damage was defined as breaks in two neighbouring elastic fibers surrounded by connective tissue (McLoughlin D et al. (2011), Circulation 124:S168).

### Animal models

Mouse models were used to prove the in vivo reproducibility of the results obtained using cell culture models. All animal experiments were carried out under the approval of the regional animal ethics committee (Regierungspräsidium Karlsruhe, permit numbers G187-11 and G-52/17, applicant Prof. Dr. Klaus Kallenbach). Animals were kept in the Interfaculty Biomedical Facility (IBF), Heidelberg under standard conditions with 12-hour light and 12-hour night cycle; water and food was offered ad libitum.

### Aortic grafts transplantation

For the Marfan model, 6-9 weeks old female mice were used. The animals express very little fibrillin-1 due to a hypomorphic mutation (mgR/mgR). Male mice were excluded because they present with a significantly lower survival in this model. As comparison age-matched wild type littermates were used.

AAV9SLR-EGFP, AAV9WT and AAV9SLR expressing the hairpin RNA decoy ODNs were applied at the level of the aorta *ex vivo* by means of a heterotopic infrarenal transplantation model. Surgeries were performed by Dr. Rawa Arif and Maximilian Franz, Clinic for Cardiac Surgery, Heidelberg University Hospi tal. Donor mgR/mgR mice were euthanized with CO₂, the thorax was opened and 5 mL of cold sterile saline solution was injected systemically through the left ventricle. The thoracic aorta was gently removed and grafts were incubated with gentle shaking on a tumbling table for 30 min with 50 µL AAV solution (10¹² virus particles/mL, diluted in NaCI) at ambient temperature (warm ischemia). In order to demonstrate that AAV transduction itself has no effect on elastin structure, mgR/mgR grafts were treated with PBS as a control. Recipient mgR/mgR mice were anesthetized with isoflurane and the abdomen was opened. The infrarenal part of the aorta was visualized under the microscope (25-40× magnification) and vessel clips were placed in between the left renal artery and the aortic bifurcation prior to cutting it. The *ex vivo* transduced aortic graft was implanted, the clips were detached and the abdomen was closed. The following 3 days after surgery, analgesic treatment (Novalgin, Camprofen) was provided. Grafts were removed for analysis 30 days post surgery.

### Statistical data analysis

The statistical data evaluation was made using GraphPad InStat 3.06 software. Differences between 3 or more different groups were assessed using One-way ANOVA followed by a Tukey's multiple comparison test for particular pairs of groups. Mann-Whitney U test was used to compare two groups. A p value <0.05 was considered significant. *In vitro* experiments were normally repeated at least 4 times. The mean fluorescence intensity of at least 20 images/group was analyzed using ImageJ (FiJi version 1.51p) when tissue sections were subjected to immunofluorescence analysis. Gel zymography and Western blot data were evaluated as well using ImageJ. Data are presented as means ± SD of n individual experiments. Graphs were generated using GraphPad Prism 7 (San Diego, California, USA).

Non-standard literature cited:
Arif et al. (2017), Nucleic Acids 9:69
Buchwald et al. (2002), J Am Coll Cardiol 39:732
Feng & Doolittle (1987), J. Mol. Evolution 25: 351
Hecker et al. (2008). Decoy oligodeoxynucleotides to treat inflammatory diseases. (book chapter) In: Therapeutic Oligonucleotides; (ed. Kurreck J) RSC Publishing, Cambridge, U.K., pp. 163
Higgins et al. (1989), CABIOS 5:151
Jensen (2013), The Anatomical Record 296:378
McLoughlin D et al. (2011), Circulation 124:S168
Needleman and Wunsch (1970), J. Mol. Biol. 48:443
Park et al. (2005), Gene Ther Mol Biol 9:15
Pyeritz RE (2017) Ann Cardiothorac Surg 6:595
Smith and Waterman (1981), Adv. Appl. Math. 2: 482-489
Varadi et al. (2012), Gene Therapy 19:800
Wile et al. (2014), Nature Protocols 9:2411

## Claims

1. An expression construct encoding a short-hairpin activator protein-1 binding polynucleotide (shAP-1 binding polynucleotide), said shAP-1 binding polynucleotide comprising an activator protein-1 (AP-1) binding site sequence and a reverse complement of said AP-1 binding site sequence separated by a spacer sequence.

2. The expression construct of claim 1, wherein said AP-1 binding site sequence has a length of from 7 to 25 nucleotides, preferably 7 to 10 nucleotides and/or wherein said shAP-1 binding polynucleotide has a length of from 30 to 70 nucleotides.

3. The expression construct of claim 1 or 2, wherein said shAP-1 binding polynucleotide is a short-hairpin RNA (shRNA).

4. The expression construct of any one of claims 1 to 3 further comprising at least one sequence mediating packaging of said expression construct into a viral particle.

5. The expression construct of any one of claims 1 to 4 further comprising an RNA polymerase promoter, preferably a cell-specific promoter, upstream of the shAP-1 binding polynucleotide encoding sequence.

6. The expression construct of any one of claims 1 to 5, wherein said AP-1 binding site sequence comprises the sequence TGASTCA (SEQ ID NO:1), with S being C or G.

7. The expression construct of any one of claims 1 to 6, wherein said AP-1 binding site sequence comprises the sequence GTGCTGACTCAGCAC (SEQ ID NO: 2); preferably comprises the sequence TGTGCTGACTCAGCACA (SEQ ID NO: 3); TTGTGCTGACTCAGCACAA (SEQ ID NO: 4); CGCTTGATGACTCAGCCGGAA (SEQ ID NO: 5),; GTCGCTTAGTGACTAAGCGAC (SEQ ID NO: 6), TTGCTGACTCATGAGTCAGCAA (SEQ ID NO: 7), CTGCGGTGCTGACTCAGCACG (SEQ ID NO: 8), CGTGCTGAGTCAGCACCGCAG (SEQ ID NO: 9), or a sequence at least 75% identical to any of the above, preferably comprising the sequence of SEQ ID NO: 1.

8. A polyribonucleotide comprising an activator protein 1 (AP-1) binding site sequence and a reverse complement of said AP-1 binding site sequence separated by a spacer sequence, preferably produced or producible from the expression construct according to any one of claims 1 to 7.

9. An expression construct according to any one of claims 1 to 7 and/or a polyribonucleotide according to claim 8 for use in treating, preventing, and/or preventing progression of disease.

10. An expression construct according to any one of claims 1 to 7 and/or a polyribonucleotide according to claim 8 for use in treating, preventing, and/or preventing progression of disease caused by extracellular matrix insufficiency and/or inflammation within the blood vessel wall.

11. The expression construct and/or polyribonucleotide for use of claim 10, wherein said disease caused by extracellular matrix insufficiency is Marfan syndrome.

12. The expression construct and/or polyribonucleotide for use of claim 10, wherein said disease caused by inflammation within the blood vessel wall is chronic graft rejection, venous bypass vasculopathy, varicose veins, and/or restenosis after percutaneous transluminal angioplasty.

13. A viral particle comprising an expression construct according to any one of claims 1 to 7 and/or a polyribonucleotide according to claim 8.

14. A composition comprising an expression construct according to any one of claims 1 to 7, a polyribonucleotide according to claim 8, and/or a viral particle according to claim 13, and a carrier.

15. Use of an expression construct according to any one of claims 1 to 7, a polyribonucleotide according to claim 8, and/or a viral particle according to claim 13 for inhibiting, preferably in vitro inhibiting, at least AP-1.


SEQUENCE LISTING

<110>  Universität Heidelberg

<120>  AP-1 binding polynucleotides

<130>  UH15068EP

<160>  12

<170>  PatentIn version 3.3

<210>  1
<211>  7
<212>  DNA
<213>  Artificial

<220>
<223>  AP-1 binding sequence 1


<220>
<221>  misc_feature
<222>  (4)..(4)
<223>  S is C or G

<400>  1
tgastca                                                                 7


<210>  2
<211>  15
<212>  DNA
<213>  Artificial

<220>
<223>  AP-1 binding sequence 2

<400>  2
gtgctgactc agcac                                                       15


<210>  3
<211>  17
<212>  DNA
<213>  Artificial

<220>
<223>  AP-1 binding sequence

<400>  3
tgtgctgact cagcaca                                                     17


<210>  4
<211>  19
<212>  DNA
<213>  Artificial

<220>
<223>  AP-1 binding sequence 4

<400>  4

ttgtgctgac tcagcacaa                                                    19


<210>  5
<211>  21
<212>  DNA
<213>  Artificial

<220>
<223>  AP-1 binding sequence 5

<400>  5
cgcttgatga ctcagccgga a                                                21


<210>  6
<211>  21
<212>  DNA
<213>  Artificial

<220>
<223>  AP-1 binding sequence 6

<400>  6
gtcgcttagt gactaagcga c                                                21


<210>  7
<211>  22
<212>  DNA
<213>  Artificial

<220>
<223>  AP-1 binding sequence 7

<400>  7
ttgctgactc atgagtcagc aa                                               22


<210>  8
<211>  21
<212>  DNA
<213>  Artificial

<220>
<223>  AP-1 binding sequence 8

<400>  8
ctgcggtgct gactcagcac g                                                21


<210>  9
<211>  21
<212>  DNA
<213>  Artificial

<220>
<223>  AP-1 binding sequence

<400>  9
cgtgctgagt cagcaccgca g                                                21

<210>  10
<211>  45
<212>  DNA
<213>  Artificial

<220>
<223>  hpAP-1 decoy ODN

<400>  10
ctgcggtgct gactcagcac gaaacgtgct gagtcagcac cgcag                    45


<210>  11
<211>  177
<212>  DNA
<213>  Artificial

<220>
<223>  hpAP-1 decoy ODN with consensus binding site

<400>  11
aggcgccctg caatatttgc atgtcgctat gtgttctggg aaatcaccat aaacgtgaaa     60

tgtctttgga tttgggaatc ttataagttc tgtatgagac cacagtcgac ctgcggtgct    120

gactcagcac gaaacgtgct gagtcagcac cgcagcaccg cagtttcgac ctcgaga       177


<210>  12
<211>  177
<212>  DNA
<213>  Artificial

<220>
<223>  hpAP-1 decoy ODN with mutated binding site

<400>  12
aggcgccctg caatatttgc atgtcgctat gtgttctggg aaatcaccat aaacgtgaaa     60

tgtctttgga tttgggaatc ttataagttc tgtatgagac cacagtcgac ctgcggtgct    120

tacttagcac gaaacgtgct aagtaagcac cgcagcaccg cagtttcgac ctcgaga       177